# EUROPEAN PATENT APPLICATION

(11) **EP 4 840 869 A1**
(43) Date of publication of application: **18.11.2026**
(21) Application number: 24916724.8
(22) Date of filing: 24.12.2024
(51) Int. Cl.: C07H 15/18, C07H 15/26, A61K 31/7048, A61K 31/7056, A61K 31/7028, A61P 9/10, A61P 27/06, A61P 27/12, A61P 27/02

(54) **COMPOUND, AND PHARMACEUTICAL COMPOSITION CONTAINING SAME AND USE THEREOF**

(30) Priority: 08.01.2024 CN 202410023412
(71) Applicant: Suzhou Raymon Pharmaceuticals Company, Ltd., Suzhou, Jiangsu 215123 (CN)
(72) Inventor: ZHANG, Fei, Suzhou, Jiangsu 215123 (CN)
(74) Representative: Müller, Christian Stefan Gerd
(86) International application number: PCT/CN2024/141723
(87) International publication number: WO 2025/148677

(57) **Abstract**

The present invention relates to a compound having the structure of formula (I) or a pharmaceutically acceptable salt thereof, and further relates to a pharmaceutical composition comprising the same, a preparation method therefor and use thereof.

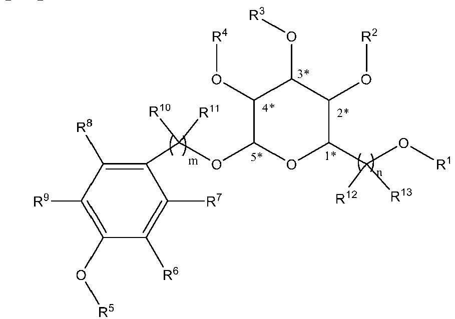

## Description

### Technical Field

The present invention relates to the field of medicinal chemistry and biomedicine, in particular, the present invention relates to a novel compound having the structure of formula (I) or a pharmaceutically acceptable salt thereof, a pharmaceutical composition comprising the same, and use thereof in the manufacture of a medicament for preventing or treating a disease of abnormal extracellular matrix proteins and a disease related to high intraocular pressure.

### Background

Glycoside compounds can play a relatively good role in anti-oxidation, anti-inflammatory, anti-ischemia-reperfusion injury, relaxation of vascular smooth muscle, nerve protection, anti-fatigue, anti-aging, immune regulation, alleviation of corneal epithelial cell oxidation damage caused by dry eye condition, anti-hepatic fibrosis, pulmonary fibrosis and renal fibrosis.

Nitric oxide (NO) is an important intercellular information transduction factor. Nitric oxide (NO) may be synthesized in vivo by nitric oxide synthase or released by drugs such as nitroglycerin. NO binds soluble guanylate cyclase (sGC) and then converts guanosine triphosphate to cyclic guanosine monophosphate (cGMP). The cGMP is a second messenger that regulates smooth muscle relaxation and vasodilation and many other important biological processes such as platelet inhibition and cell growth and differentiation. Nitric oxide has an important physiological role in regulating the blood flow of the optic nerve head and the intraocular pressure (IOP). In the optic nerve head, NO donors reduce vascular resistance by relaxing smooth muscle, leading to local vasodilation and an increase in blood flow to the optic nerve head. In contrast, the impaired NO pathway reduces the blood flow in the optic nerve head, thus leading to optic nerve damage caused by ischemia.

Glaucoma is a group of eye diseases that share the common characteristics of retinal ganglion cell degeneration, specific optic nerve atrophy, and progressive visual field loss, and is mainly associated with the increase of pathological intraocular pressure, and is the most common cause of irreversible blindness. The signal pathways related to the pathogenesis of glaucoma are extremely complex, and downstream signal factors often intersect with each other. However, they mainly play a regulatory role in the occurrence and development of glaucoma by upregulating the expression of pro-apoptotic genes, downregulating neuroprotective and regenerative factors, and promoting trabecular meshwork fibrosis. This leads to the obstruction of aqueous humor drainage and ultimately results in glaucoma. Currently, the drugs for the clinical treatment of primary open-angle glaucoma mainly achieve the purpose of reducing the intraocular pressure by decreasing the production of aqueous humor and/or promoting the drainage of aqueous humor. None of these drugs directly target the pathological mechanism of the aqueous humor outflow obstruction in the trabecular meshwork of glaucoma. In other words, they only treat the symptoms rather than the root cause. Therefore, there is an urgent need for a drug that can improve trabecular meshwork fibrosis while also reducing the intraocular pressure, so as to achieve the goal of treating both the symptoms and the root cause.

CN109045049A discloses the use of a glycoside compound in the preparation of a medicament as an inhibitor for treating and preventing diseases associated with abnormal extracellular matrix proteins such as ocular fibrosis, and high intraocular pressure.

### Summary

In a first aspect, provided is a compound having the following structure of formula (I): 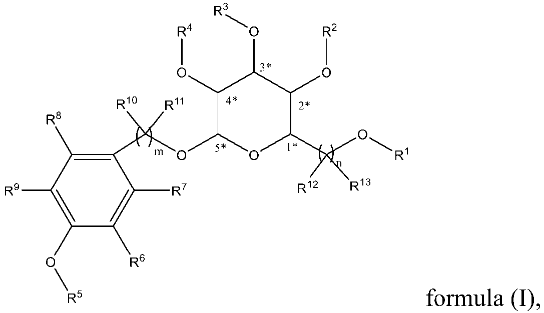
or a pharmaceutically acceptable salt thereof,
wherein,
R¹, R², R³, R⁴ and R⁵ are each independently selected from the group consisting of hydrogen, C₁₋₆ alkyl, C₂₋₆ alkenyl, C₂₋₆ alkynyl, acyl, -NO₂ and -L-R', wherein each alkyl, alkenyl, alkynyl is unsubstituted or substituted with at least one substituent independently selected from the group consisting of R^{X};
-L- is -(CH₂)ₒ-, wherein each -(CH₂)- is independently and optionally replaced by carbonyl or -O-;
R' is selected from the group consisting of hydrogen, C₁₋₆ alkyl, C₂₋₆ alkenyl, C₂₋₆ alkynyl, NO₂, 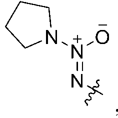 , 3-10-membered heterocycloalkyl, 5-10-membered heteroaryl, wherein each alkyl, alkenyl, alkynyl, heterocycloalkyl, heteroaryl is unsubstituted or substituted with at least one substituent independently selected from the group consisting of R^{X};
wherein at least one of R¹, R², R³, R⁴, R⁵ and R' is NO₂;
R⁶, R⁷, R⁸ and R⁹ are each independently selected from the group consisting of hydrogen, C₁₋₆ alkyl, C₁₋₆ alkyl-O-C₁₋₆ alkyl, and C₁₋₆ alkyl-S-C₁₋₆ alkyl, wherein each alkyl is unsubstituted or substituted with at least one substituent independently selected from the group consisting of R^{X};
R¹⁰, R¹¹, R¹² and R¹³ are each independently selected from the group consisting of hydrogen, C₁₋₆ alkyl, C₁₋₆ alkyl-O-C₁₋₆ alkyl, and C₁₋₆ alkyl-S-C₁₋₆ alkyl, wherein each alkyl is unsubstituted or substituted with at least one substituent independently selected from the group consisting of R^{X};
R^{X} is selected from the group consisting of hydroxyl, halogen, cyano, C₁₋₆ alkyl, C₁₋₆ alkoxy, C₂₋₆ alkenyl, C₂₋₆ alkynyl and 5-10-membered heteroaryl, wherein each hydroxyl, alkyl, alkoxy, alkenyl, alkynyl, heteroaryl is unsubstituted or substituted with at least one substituent independently selected from the group consisting of R^{Y};
R^{Y} is selected from the group consisting of hydroxyl, halogen, cyano, NO₂, C₁₋₆ alkyl, C₁₋₆ alkoxy, C₂₋₆ alkenyl and C₂₋₆ alkynyl;
^{1*}C, ^{2*}C, ^{3*}C, ^{4*}C and ^{5*}C respectively represent the carbon atoms which may have chirality in the compound having the structure of formula (I);
o, m, and n are each independently selected from the group consisting of an integer of 0-6.

In a second aspect, provided is a pharmaceutical composition comprising a compound of formula (I) of the present invention or a pharmaceutically acceptable salt thereof and at least one pharmaceutically acceptable carrier.

In a third aspect, provided is use of a compound of formula (I) or a pharmaceutically acceptable salt thereof in the manufacture of a medicament for treating, ameliorating or preventing a disease of abnormal extracellular matrix proteins, such as ocular fibrosis, and a disease related to high intraocular pressure.

### Brief Description of Drawings

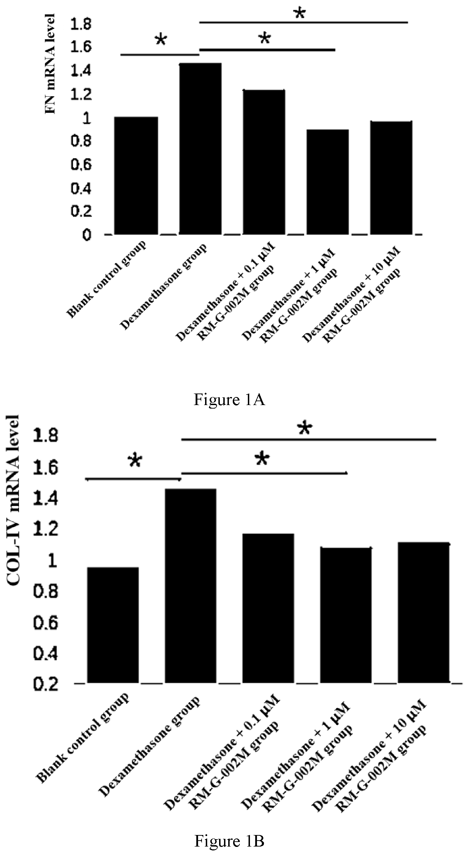

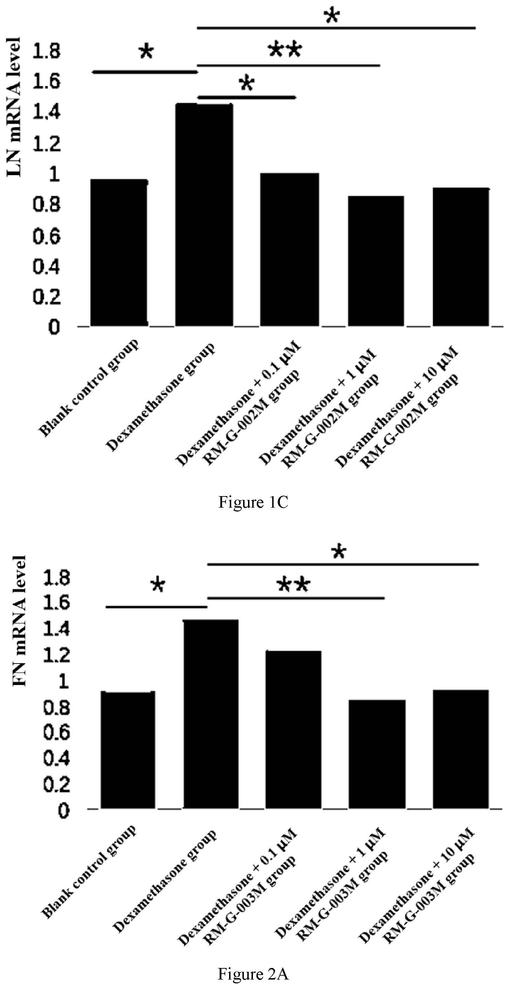

Figures 1A to 1C show the effect of compound RM-G-002M of the present invention at different concentrations on the human trabecular meshwork extracellular matrix induced by dexamethasone. Figure 1A shows the effect of compound RM-G-002M of the present invention at different concentrations on the level of fibronectin (FN) mRNA; Figure 1B shows the effect of compound RM-G-002M of the present invention at different concentrations on the level of collagen type IV (COL-IV) mRNA; and Figure 1C shows the effect of compound RM-G-002M of the present invention at different concentrations on the level of laminin (LN) mRNA.

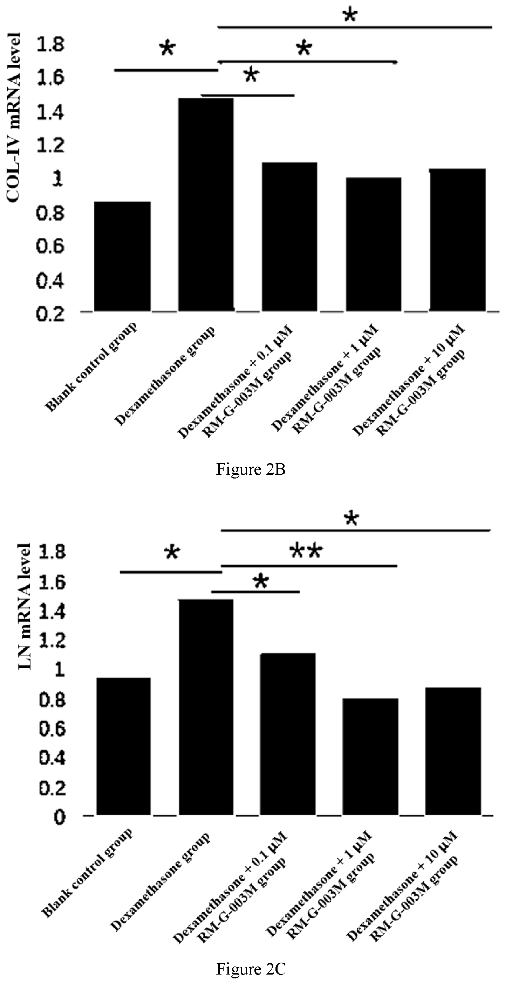

Figures 2A to 2C show the effect of compound RM-G-003M of the present invention at different concentrations on the human trabecular meshwork extracellular matrix induced by dexamethasone. Figure 2A shows the effect of compound RM-G-003M of the present invention at different concentrations on the level of FN mRNA; Figure 2B shows the effect of compound RM-G-003M of the present invention at different concentrations on the level of COL-IV mRNA; Figure 2C shows the effect of compound RM-G-003M of the present invention at different concentrations on the level of LN mRNA.

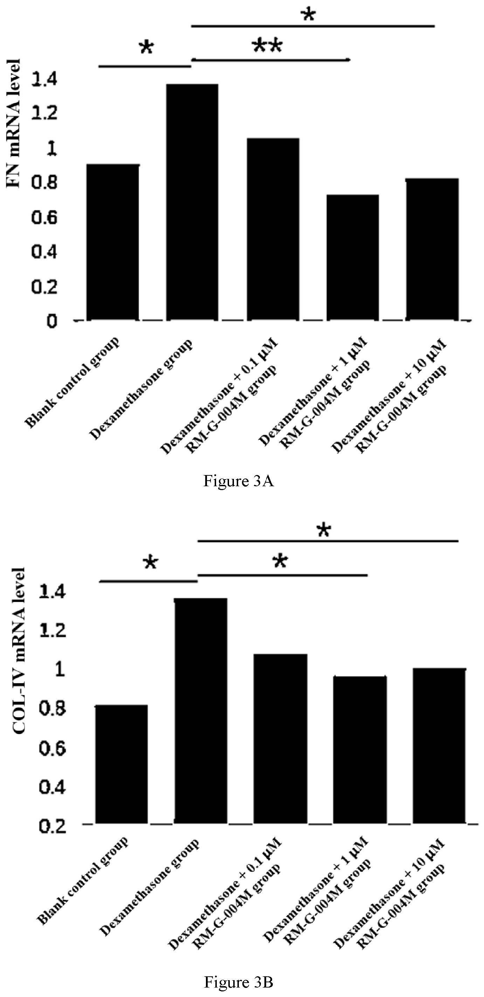

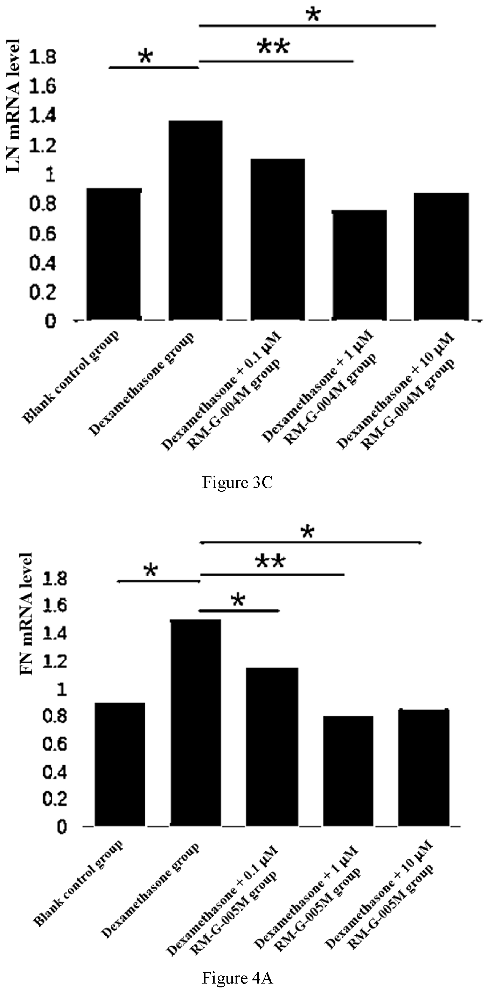

Figures 3A to 3C show the effect of compound RM-G-004M of the present invention at different concentrations on the human trabecular meshwork extracellular matrix induced by dexamethasone. Figure 3A shows the effect of compound RM-G-004M of the present invention at different concentrations on the level of FN mRNA; Figure 3B shows the effect of compound RM-G-004M of the present invention at different concentrations on the level of COL-IV mRNA; Figure 3C shows the effect of compound RM-G-004M of the present invention at different concentrations on the level of LN mRNA.

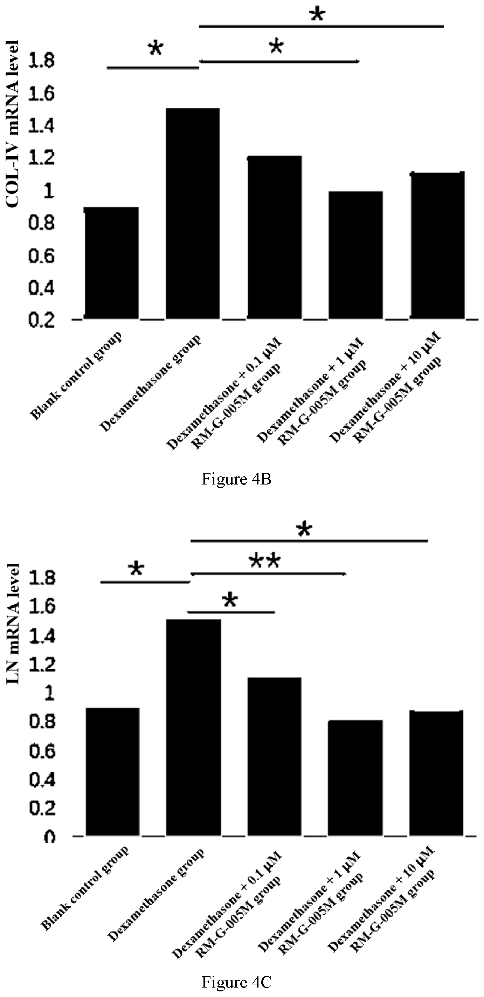

Figures 4A to 4C show the effect of compound RM-G-005M of the present invention at different concentrations on the human trabecular meshwork extracellular matrix induced by dexamethasone. Figure 4A shows the effect of compound RM-G-005M of the present invention at different concentrations on the level of FN mRNA; Figure 4B shows the effect of compound RM-G-005M of the present invention at different concentrations on the level of COL-IV mRNA; Figure 4C shows the effect of compound RM-G-005M of the present invention at different concentrations on the level of LN mRNA.

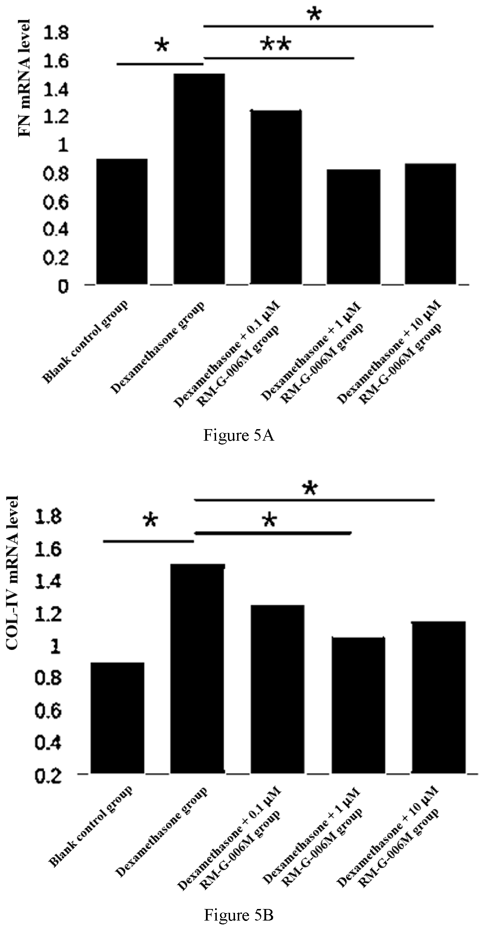

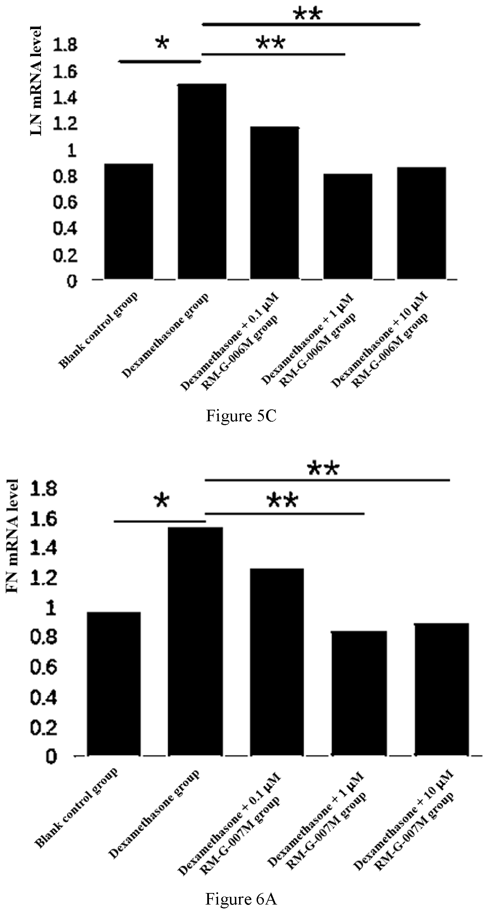

Figures 5A to 5C show the effect of compound RM-G-006M of the present invention at different concentrations on the human trabecular meshwork extracellular matrix induced by dexamethasone. Figure 5A shows the effect of compound RM-G-006M of the present invention at different concentrations on the level of FN mRNA; Figure 5B shows the effect of compound RM-G-006M of the present invention at different concentrations on the level of COL-IV mRNA; Figure 5C shows the effect of compound RM-G-006M of the present invention at different concentrations on the level of LN mRNA.

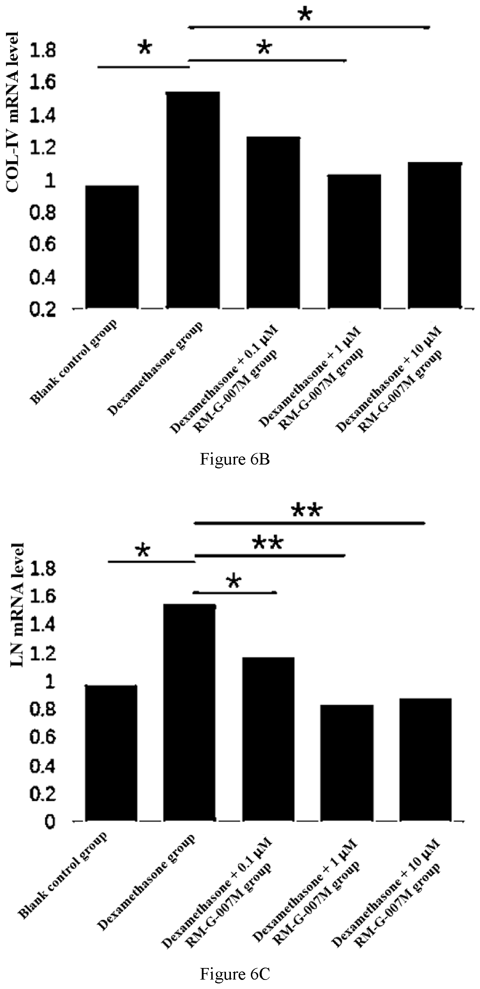

Figures 6A to 6C show the effect of compound RM-G-007M of the present invention at different concentrations on the human trabecular meshwork extracellular matrix induced by dexamethasone. Figure 6A shows the effect of compound RM-G-007M of the present invention at different concentrations on the level of FN mRNA; Figure 6B shows the effect of compound RM-G-007M of the present invention at different concentrations on the level of COL-IV mRNA; Figure 6C shows the effect of compound RM-G- 007M of the present invention at different concentrations on the level of LN mRNA.

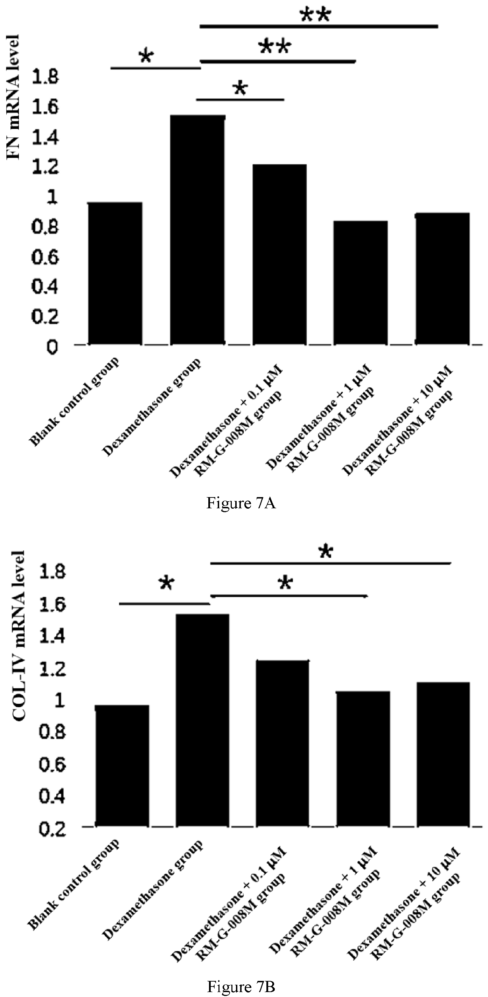

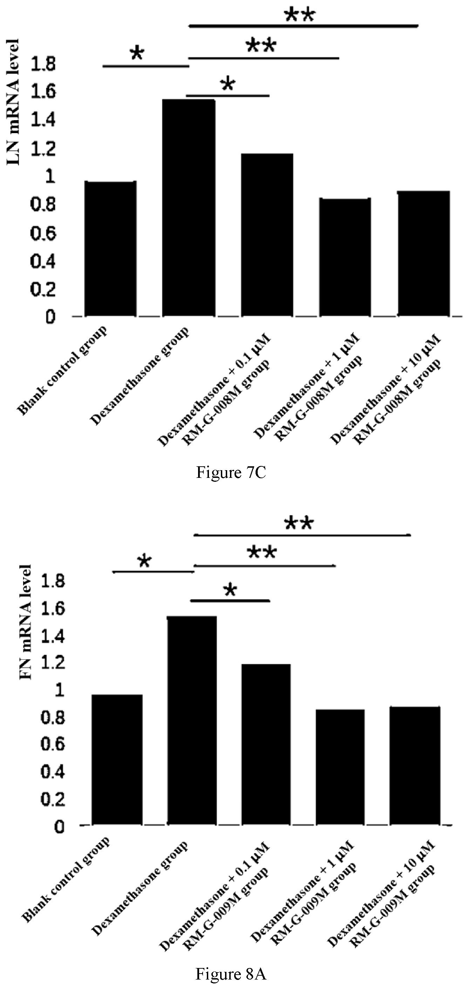

Figures 7A to 7C show the effect of compound RM-G-008M of the present invention at different concentrations on the human trabecular meshwork extracellular matrix induced by dexamethasone. Figure 7A shows the effect of compound RM-G-008M of the present invention at different concentrations on the level of FN mRNA; Figure 7B shows the effect of compound RM-G-008M of the present invention at different concentrations on the level of COL-IV mRNA; Figure 7C shows the effect of compound RM-G-008M of the present invention at different concentrations on the level of LN mRNA.

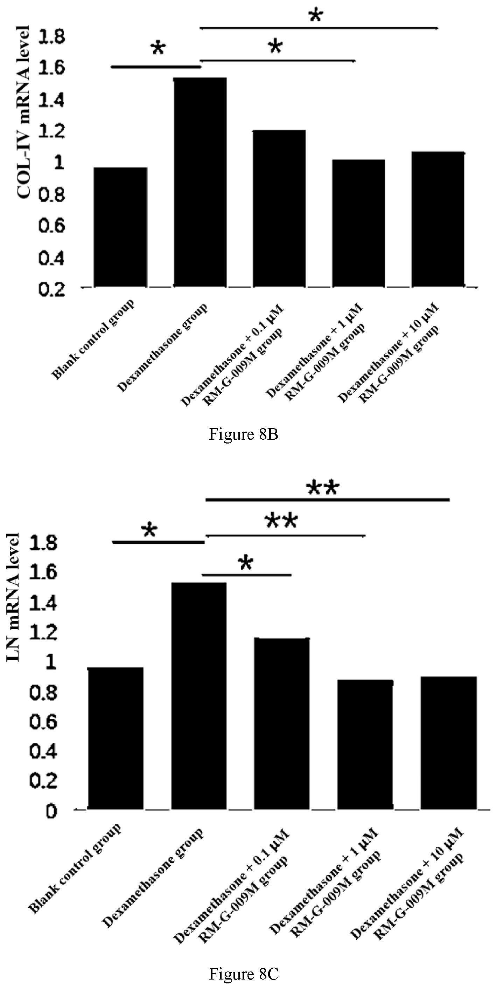

Figures 8A to 8C show the effect of compound RM-G-009M of the present invention at different concentrations on the human trabecular meshwork extracellular matrix induced by dexamethasone. Figure 8A shows the effect of compound RM-G-009M of the present invention at different concentrations on the level of FN mRNA; Figure 8B shows the effect of compound RM-G-009M of the present invention at different concentrations on the level of COL-IV mRNA; Figure 8C shows the effect of compound RM-G-009M of the present invention at different concentrations on the level of LN mRNA.

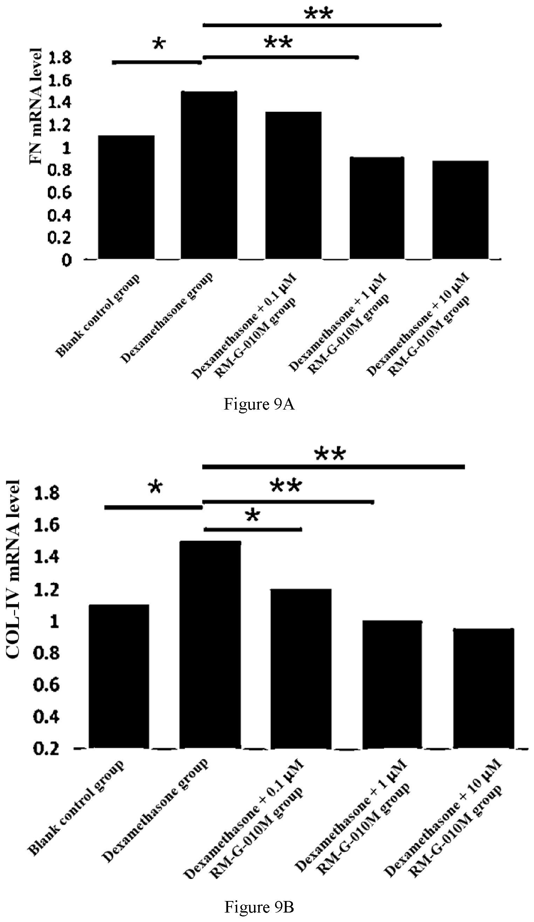

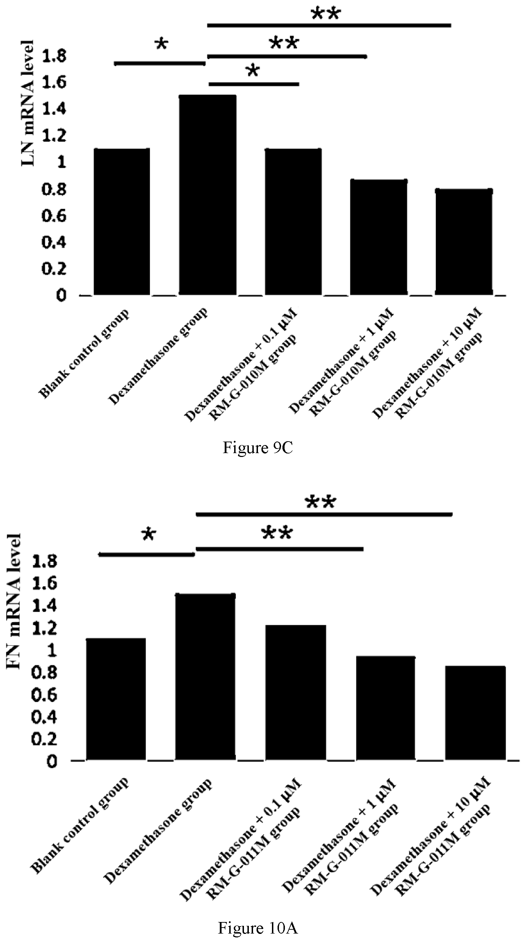

Figures 9A to 9C show the effect of compound RM-G-010M of the present invention at different concentrations on the human trabecular meshwork extracellular matrix induced by dexamethasone. Figure 9A shows the effect of compound RM-G-010M of the present invention at different concentrations on the level of FN mRNA; Figure 9B shows the effect of compound RM-G-010M of the present invention at different concentrations on the level of COL-IV mRNA; Figure 9C shows the effect of compound RM-G-010M of the present invention at different concentrations on the level of LN mRNA.

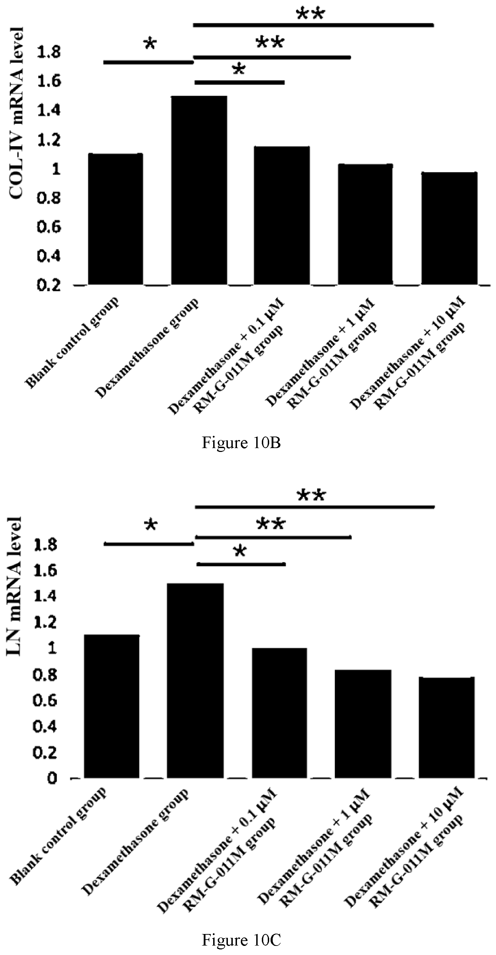

Figures 10A to 10C show the effect of compound RM-G-011M of the present invention at different concentrations on the human trabecular meshwork extracellular matrix induced by dexamethasone. Figure 10A shows the effect of compound RM-G-011M of the present invention at different concentrations on the level of FN mRNA; Figure 10B shows the effect of compound RM-G-011M of the present invention at different concentrations on the level of COL-IV mRNA; Figure 10C shows the effect of compound RM-G-011M of the present invention at different concentrations on the level of LN mRNA.

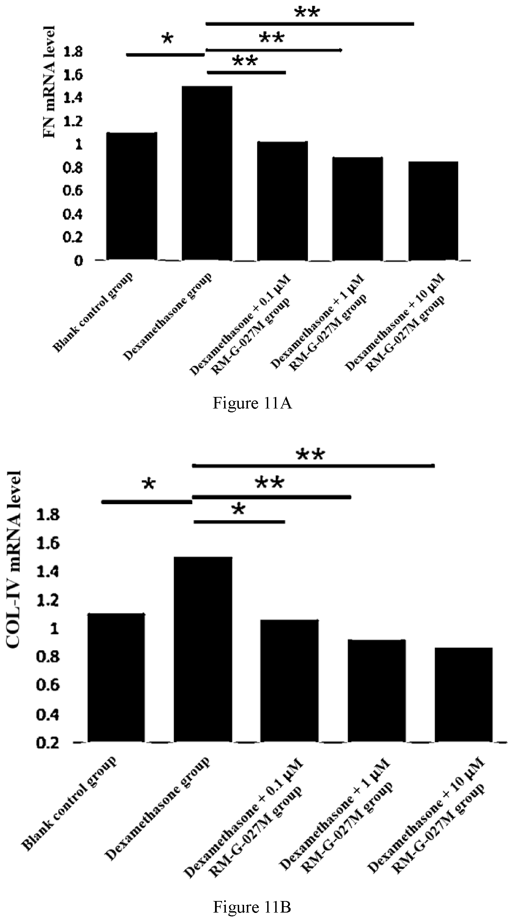

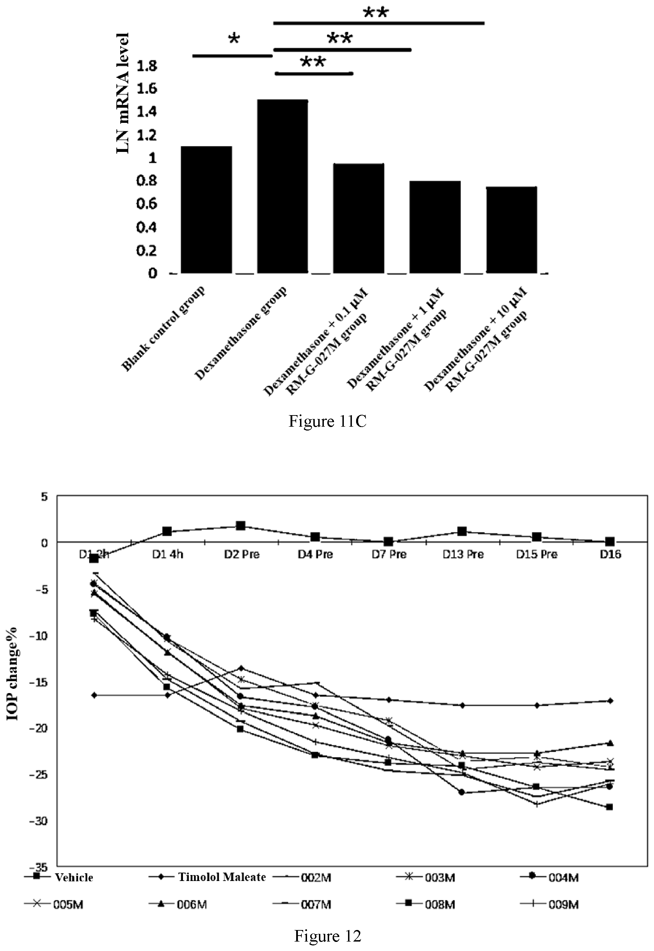

Figures 11A to 11C show the effect of compound RM-G-027M of the present invention at different concentrations on the human trabecular meshwork extracellular matrix induced by dexamethasone. Figure 11A shows the effect of compound RM-G-027M of the present invention at different concentrations on the level of FN mRNA; Figure 11B shows the effect of compound RM-G-027M of the present invention at different concentrations on the level of COL-IV mRNA; Figure 11C shows the effect of compound RM-G-027M of the present invention at different concentrations on the level of LN mRNA.
Figure 12 shows the effect of compounds RM-G-002M to RM-G-009M of the present invention on the intraocular pressure of beagle dogs.

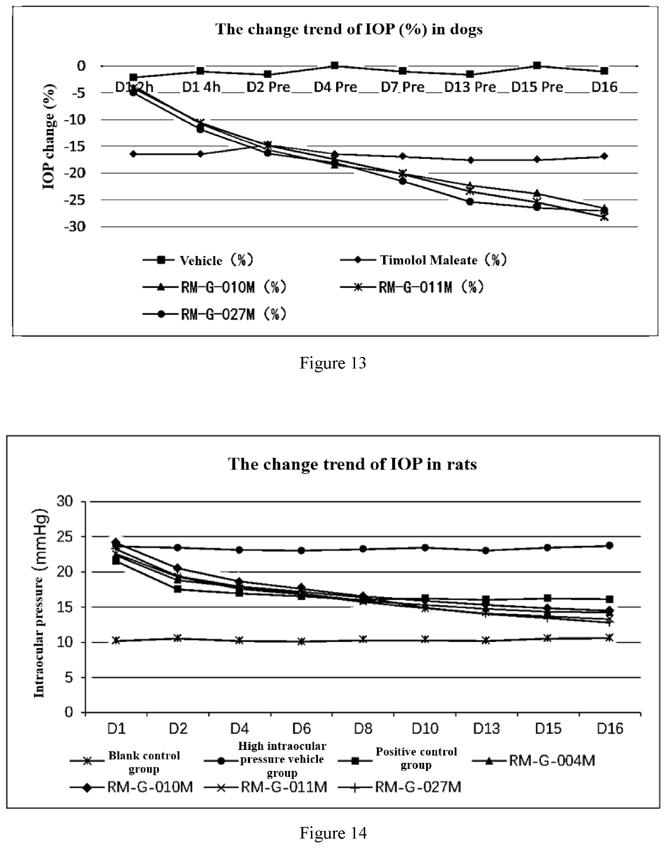

Figure 13 shows the effect of compounds RM-G-010M, RM-G-011M and RM-G-027M of the present invention on the intraocular pressure of beagle dogs.
Figure 14 shows the effect of compounds RM-G-004M, RM-G-010M, RM-G-011M and RM-G-027M of the present invention on the intraocular pressure of rats.

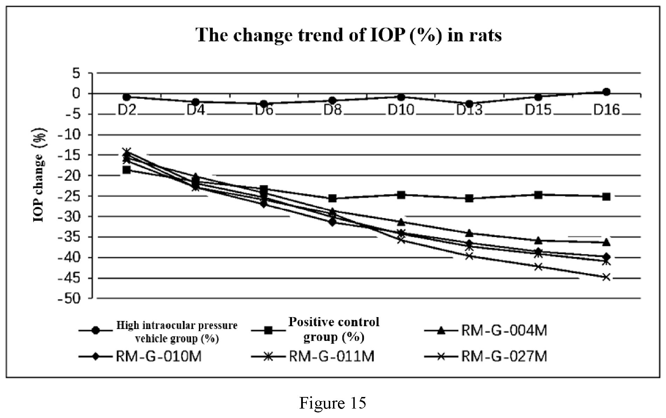

Figure 15 shows the effect of compounds RM-G-004M, RM-G-010M, RM-G-011M and RM-G-027M of the present invention on the intraocular pressure of rats.

### Detailed Description

The present invention will be further described in detail below. Such description is for illustrative purposes and is not limiting. Those skilled in the art can easily understand other advantages and effects of the present disclosure from the content disclosed in the specification. The invention may also be implemented or applied in other different embodiments. Those skilled in the art can make various modifications and variations without departing from the spirit of the present disclosure.

### General Terms and Definitions

Unless defined otherwise below, all technical and scientific terms used herein have the same meaning as commonly understood by one of ordinary skill in the art. In case of any contradiction, the definitions provided herein shall prevail. The technology used herein refers to those generally understood in the art, including variations and equivalents that will be apparent to those skilled in the art. While the following terms are believed to be readily understood by those skilled in the art, the following definitions are set forth to better illustrate the invention. When a trade name appears herein, it refers to the corresponding commercial products or its active ingredient. All patents, published patent applications, and publications cited herein are incorporated herein by reference.

When an amount, concentration, or other value or parameter is set forth in the form of a range, preferred range or upper or lower preferred limit, this is to be understood as being equivalent to specifically disclosing any range formed by combining any upper limit or preferred value with any lower limit or preferred value, regardless of whether the range is explicitly recited. Unless otherwise indicated, the numerical ranges listed herein are intended to include the endpoints of the ranges as well as all integers and fractions (decimal) within the ranges. For example, the expression "m is an integer of 0-6" means that m is any integer of 0-6, for example, m may be 0, 1, 2, 3, 4, 5 or 6. Other similar expressions such as n and o should also be understood in a similar manner.

The singular forms "a," "an," and "the" include plural forms unless the context clearly dictates otherwise. The expression "one or more" or "at least one" may mean 1, 2, 3, 4, 5, 6, 7, 8, 9 or more.

The terms "about" and "approximately," when used with numerical variables, generally mean that the value of the variable and all values of the variable are within experimental error (e.g., within a 95% confidence interval of the mean value) or within ± 10% or wider of the specified value.

The expressions "comprise", "include", "contain" and "have" are open-ended and do not exclude additional unlisted elements, steps or components. The expression "consisting of..." does not include any unspecified element, step or ingredient. The expression "consisting essentially of..." means that the scope is limited to the specified elements, steps or components, as well as the elements, steps or components that optionally exist and do not substantially affect the essential and novel features of the claimed subject matter. It should be understood that the expression "comprising" includes the expressions "consisting essentially of..." and "consisting of...".

The term "alkyl" refers to a straight or branched saturated aliphatic hydrocarbon group composed of carbon atoms and hydrogen atoms, which is attached to the remainder of the molecule via a single bond. The alkyl group may have 1-20 carbon atoms, referring to "C ₁₋₂₀ alkyl", for example, C ₁₋₆ alkyl, C ₁₋₄ alkyl, C ₁₋₂ alkyl, C ₃ alkyl, C ₄ alkyl, or C ₃₋₆ alkyl. "C ₁₋₆ alkyl" is used to denote a linear or branched saturated hydrocarbon group consisting of 1 to 6 carbon atoms. The C ₁₋₆ alkyl includes C ₁₋₅ alkyl, C ₁₋₄ alkyl, C ₁₋₃ alkyl, C ₁₋₂ alkyl, C ₂₋₆ alkyl, C ₂₋₄ alkyl, C ₆ alkyl, and C ₅ alkyl, etc. Non-limiting examples of alkyl groups include, but are not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or isomers thereof.

A divalent group refers to a group obtained by removal of one hydrogen atom from a carbon atom with a free valence electron of a corresponding monovalent group. The divalent group has two attachment sites linked to the remainder of the molecule. For example, "alkylene" or "alkylidene" refers to a saturated linear or branched divalent hydrocarbon group. Examples of "alkylene" include, but are not limited to, methylene (-CH ₂-), ethylene (-C ₂H ₄-), propylene (-C ₃H ₆-), butylene (-C ₄H ₈-), pentylene (-C ₅H ₁₀-), hexylene (-C ₆H ₁₂-), 1-methylethylene (-CH(CH ₃)CH ₂-), 2-methylethylene (-CH ₂CH(CH ₃)-), methylpropylene or ethylpropylene, etc. For example, -(CH₂)ₒ- as used herein is a divalent group.

Unless otherwise specified, "cycloalkyl" includes any stable cyclic alkyl group, including monocyclic, bicyclic, or tricyclic systems, wherein bicyclic and tricyclic systems include spiro, fused, and bridged rings. It can be monosubstituted or polysubstituted, and can be monovalent, divalent or multivalent. Examples of these cycloalkyl include, but are not limited to, cyclopropyl, cyclobutyl, cyclopentyl, cyclohexyl, cycloheptyl, norbornyl, [2.2.2] bicyclooctane, [4.4.0] bicyclodecane, etc.

Unless otherwise specified, the term "heterocycloalkyl," by itself or in combination with other terms, means a cyclic "heteroalkyl," which includes monocyclic, bicyclic, and tricyclic systems, wherein bicyclic and tricyclic systems include spiro, fused, and bridged rings. In addition, with respect to the "heterocycloalkyl", the heteroatom may occupy the position at which the heterocycloalkyl is linked to the remainder of the molecule. In some embodiments, the heterocycloalkyl is a 3-10 membered heterocycloalkyl; in other embodiments, the heterocycloalkyl is a 5-8 membered heterocycloalkyl. Examples of heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl and tetrahydrothien-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxanyl, dithianyl, isoxazolidinyl, isothiazolidinyl, 1,2 -oxazinyl, 1,2-thiazinyl, hexahydropyridazinyl, homopiperazinyl, homopiperidinyl, or oxepanyl, hexahydrofuro [3,2 -b] furanyl.

The term "alkenyl" is used to denote a straight or branched hydrocarbon group containing one or more carbon-carbon double bonds, which may be located anywhere on the group. Non-limiting examples of alkenyl include, but are not limited to, ethenyl, propenyl, butenyl, pentenyl, hexenyl, butadienyl, pentadienyl, hexadienyl, etc.

The term "alkynyl" is used to denote a straight or branched hydrocarbon group containing one or more carbon-carbon triple bonds, which may be located anywhere on the group. Non-limiting examples of alkynyl include, but are not limited to, ethynyl, propynyl, butynyl, pentynyl, etc.

The term "alkoxy" refers to an alkyl group having a specified number of carbon atoms linked via an oxygen bridge. Non-limiting examples of alkoxy include, but are not limited to, methoxy, ethoxy, n-propoxy, isopropoxy, n-butoxy, sec-butoxy, tert-butoxy, and n-pentoxy. The term "C ₁₋₆ alkoxy" refers to an alkyl group containing 1 to 6 carbon atoms attached to the remainder of the molecule via an oxygen atom. The C ₁₋₆ alkoxy includes C ₁₋₄, C ₁₋₃, C ₁₋₂, C ₂₋₆, C ₂₋₄, C ₆, C ₅, C ₄ and C ₃ alkoxy, etc. Non-limiting examples of C₁₋₆ alkoxy include, but are not limited to, methoxy, ethoxy, propoxy (including n-propoxy and isopropoxy), butoxy (including n-butoxy, isobutoxy, s-butoxy and t-butoxy), pentoxy (including n-pentoxy, isopentoxy and neopentoxy), hexyloxy, etc.

Unless otherwise specified, the terms "aromatic ring" and "aryl" may be used interchangeably, and the term "aromatic ring" or "aryl" refers to a polyunsaturated carbocyclic ring system, which may be a monocyclic, bicyclic, or polycyclic ring system in which at least one ring is aromatic and the rings in the bicyclic and polycyclic ring systems are fused together. It can be monosubstituted or polysubstituted, and can be monovalent, divalent or multivalent. Examples of aryl include, but are not limited to, phenyl, naphthyl (including 1-naphthyl and 2-naphthyl, etc.).

Unless otherwise specified, the terms "heteroaromatic ring" and "heteroaryl" as used herein may be used interchangeably. The term "heteroaryl" refers to an aryl group (or aromatic ring) containing 1, 2, 3, or 4 heteroatoms independently selected from the group consisting of B, N, O, and S, which may be a monocyclic, bicyclic, or tricyclic ring system, wherein the nitrogen atom may be substituted or unsubstituted (i.e., N or NR, wherein R is H or other substituents already defined herein), and optionally quaternized. The nitrogen and sulfur heteroatoms may optionally be oxidized (i.e., NO and S(O)ₚ, p is 1 or 2). The heteroaryl group may be attached to the remainder of the molecule via a heteroatom. In some embodiments, the heteroaryl is 5-10-membered heteroaryl; in other embodiments, the heteroaryl is 5-6-membered heteroaryl. Examples of the heteroaryl include, but are not limited to, pyrrolyl (including N-pyrrolyl, 2-pyrrolyl and 3-pyrrolyl, and the like), pyrazolyl (including 2-pyrazolyl and 3-pyrazolyl, and the like), imidazolyl (including N-imidazolyl, 2-imidazolyl, 4-imidazolyl and 5-imidazolyl, and the like), oxazolyl (including 2-oxazolyl, 4-oxazolyl and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like), pyridyl (including 2-pyridyl, 3-pyridyl and 4-pyridyl, and the like), pyrazinyl, pyrimidinyl (including 2-pyrimidinyl and 4-pyrimidinyl, and the like), benzothiazolyl (including 5-benzothiazolyl, and the like), purinyl, benzimidazolyl (including 2-benzimidazolyl, and the like), indolyl (including 5-indolyl, and the like), isoquinolyl (including 1-isoquinolyl and 5-isoquinolyl, and the like), quinoxalyl (including 2-quinoxalyl and 5-quinoxalyl, and the like), quinolinyl (including 3-quinolinyl and 6-quinolinyl, and the like), pyrazinyl, purinyl, phenyloxazolyl, and 1,2,5-diazole-2-oxide group.

As used herein, the term "pharmaceutically acceptable salt" refers to a pharmaceutically acceptable organic or inorganic salt. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannin, pantothenate, bitartrate, ascorbate, succinate, maleate, fumarate, gluconate, glucuronate, onic acid salt, formate, benzoate, glutamate, methanesulfonate, ethanesulfonate, benzenesulfonate, p-toluenesulfonate, and pamoate (i.e., 1-1-methylene-bis(2-hydroxy-3-naphthoate)). The compounds used in the present invention can form pharmaceutically acceptable salts with various amino acids. Suitable base salts include, but are not limited to, aluminum salts, calcium salts, lithium salts, magnesium salts, potassium salts, sodium salts, zinc salts, bismuth salts, and diethanolamine salts. A review of pharmaceutically acceptable salts is found in Handbook of Pharmaceutical Salts: Properties, Selection, and Use (P. Heinrich Stahl and Camille G. Wermuth, ed., Wiley-VCH, 2002).

"Optional" or "optionally" means that the subsequently described event or circumstance may but does not necessarily occur, and that the description includes instances where the event or circumstance occurs and instances where it does not.

The term "substituted" means that any one or more hydrogen atoms on a specific atom are substituted by a substituent, and may include deuterium and hydrogen variants, as long as the valence of the specific atom is normal and the substituted compound is stable. When a substituent is oxygen (i.e., =O), it means that two hydrogen atoms are replaced. The term "optionally substituted" means that it may or may not be substituted, and unless otherwise specified, the type and number of substituents may be arbitrary on the basis of being chemically achievable.

The compounds of the present invention may exist in specific geometric or stereoisomeric forms. The present invention contemplates all such compounds, including cis and trans isomers, (-)- and (+)-enantiomers, (R)- and (S)- enantiomers, diastereomers, (D)-isomers, (L)-isomers, racemic mixtures and other mixtures thereof, such as enantiomerically or diastereomerically enriched mixtures, all of which are within the scope of the present invention. Additional asymmetric carbon atoms may be present in substituents such as alkyl groups. All such isomers and mixtures thereof are included within the scope of the present invention.

Unless otherwise indicated, the terms "enantiomer" or "optical isomer" refer to stereoisomers that are mirror images of each other.

Unless otherwise indicated, the term "cis-trans isomer" or "geometric isomer" is caused by the inability of a double bond or a single bond of ring-forming carbon atom to rotate freely.

Unless otherwise indicated, the term "diastereomer" refers to stereoisomers of molecules that have two or more chiral centers and are non-mirror images of each other.

Unless otherwise indicated, *"(D)"* or "(+)" indicates dextrorotation, *"(L)"* or "(-)" indicates levorotation, and *"(DL)"* or "(±)" indicates racemization.

Unless otherwise indicated, a wedge-shaped solid line bond( 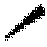 ) and a wedge-shaped dashed line bond( 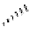 ) are used to represent an absolute configuration of a stereocenter, a straight solid line bond( 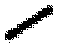 ) and a straight dashed line bond( 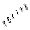 ) are used to represent a relative configuration of a stereocenter, a wavy line ( 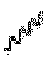 ) is used to represent the wedge-shaped solid line bond( 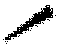 ) or the wedge-shaped dashed line bond( 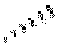 ), or a wavy line ( 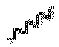 ) is used to represent the straight solid line bond( 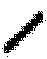 ) and the straight dashed line bond( 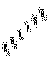 ).

The term "pharmaceutically acceptable excipient" refers to those carrier substances that have no obvious stimulating effect on the organism and do not impair the biological activity and performance of the active compound. "Pharmaceutically acceptable excipients" include, but are not limited to, glidants, sweeteners, diluents, preservatives, dyes/colorants, flavorants, surfactants, wetting agents, dispersants, disintegrants, stabilizers, solvents or emulsifiers.

The term "pharmaceutical composition" or "pharmaceutical active ingredient composition" refers to a substance consisting of one or more active ingredients and optionally one or more pharmaceutically acceptable excipients.

### Compounds of the present invention

Provided is a compound having the following structure of formula (I): 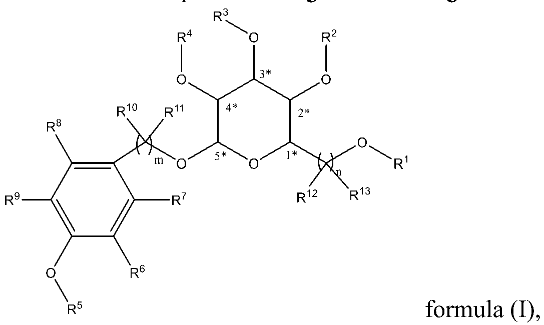
or a pharmaceutically acceptable salt thereof,
wherein,
R¹, R², R³, R⁴ and R⁵ are each independently selected from the group consisting of hydrogen, C₁₋₆ alkyl, C₂₋₆ alkenyl, C₂₋₆ alkynyl, acyl, -NO₂ and -L-R', wherein each alkyl, alkenyl, alkynyl is unsubstituted or substituted with at least one substituent independently selected from the group consisting of R^{X};
-L- is -(CH₂)ₒ-, wherein each -(CH₂)- is independently and optionally replaced by carbonyl or -O-;
R' is selected from the group consisting of hydrogen, C₁₋₆ alkyl, C₂₋₆ alkenyl, C₂₋₆ alkynyl, NO₂, 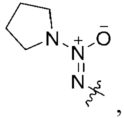 3-10-membered heterocycloalkyl, 5-10-membered heteroaryl, wherein each alkyl, alkenyl, alkynyl, heterocycloalkyl, heteroaryl is unsubstituted or substituted with at least one substituent independently selected from the group consisting of R^{X};
wherein at least one of R¹, R², R³, R⁴, R⁵ and R' is NO₂;
R⁶, R⁷, R⁸ and R⁹ are each independently selected from the group consisting of hydrogen, C₁₋₆ alkyl, C₁₋₆ alkyl-O-C₁₋₆ alkyl, and C₁₋₆ alkyl-S-C₁₋₆ alkyl, wherein each alkyl is unsubstituted or substituted with at least one substituent independently selected from the group consisting of R^{X};
R¹⁰, R¹¹, R¹² and R¹³ are each independently selected from the group consisting of hydrogen, C₁₋₆ alkyl, C₁₋₆ alkyl-O-C₁₋₆ alkyl, and C₁₋₆ alkyl-S-C₁₋₆ alkyl, wherein each alkyl is unsubstituted or substituted with at least one substituent independently selected from the group consisting of R^{X};
R^{X} is selected from the group consisting of hydroxyl, halogen, cyano, C₁₋₆ alkyl, C₁₋₆ alkoxy, C₂₋₆ alkenyl, C₂₋₆ alkynyl and 5-10-membered heteroaryl, wherein each hydroxyl, alkyl, alkoxy, alkenyl, alkynyl, heteroaryl is unsubstituted or substituted with at least one substituent independently selected from the group consisting of R^{Y};
R^{Y} is selected from the group consisting of hydroxyl, halogen, cyano, NO₂, C₁₋₆ alkyl, C₁₋₆ alkoxy, C₂₋₆ alkenyl and C₂₋₆ alkynyl;
^{1*}C, ^{2*}C, ^{3*}C, ^{4*}C and ^{5*}C respectively represent the carbon atoms which may have chirality in the compound having the structure of formula (I);
o, m, and n are each independently selected from the group consisting of an integer of 0-6.

In an embodiment, R¹ is selected from the group consisting of -NO₂ and -L-R'. In a preferred embodiment, R¹ is -NO₂. In another preferred embodiment, R¹ is -L-R', wherein -L-is -(CH₂)ₒ-, where o is 4, one end of -(CH₂)ₒ- is replaced by carbonyl, the other end of -(CH₂)ₒ- is replaced by -O-, and R' is -NO₂ and is connected to -O-. In a specific embodiment, R¹ is 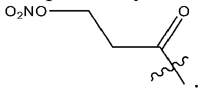 In a preferred embodiment, R¹ is -L-R', wherein -L- is -(CH₂) ₒ-, where o is 3, one end of -(CH₂)ₒ- is replaced by carbonyl, the other end of -(CH₂)ₒ- is replaced by -O-, and R' is C ₁₋₆ alkyl and is connected to -O-, which is further substituted with R ^{X}, where R ^{X} is 5-10 membered heteroaryl substituted with R^{Y}, and R^{Y} is cyano. In a specific embodiment, R¹ is 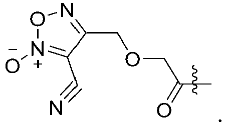 In a preferred embodiment, R¹ is -L-R', wherein -L- is -(CH₂)ₒ-, where o is 3, one end of -(CH₂)ₒ- is replaced by carbonyl, the other end of -(CH₂)ₒ- is replaced by -O-, and R' is 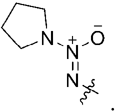 In a specific embodiment, R¹ is 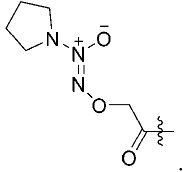 In a preferred embodiment, R¹ is -L-R', wherein -L- is -(CH₂)ₒ-, wherein o is 2, and -(CH₂)ₒ- is replaced by carbonyl and -O-, respectively, R' is 3-10 membered heterocycloalkyl and is connected to -O-, which is further substituted with R^{X}, where R^{X} is hydroxyl substituted with R^{Y}, and R^{Y} is NO₂. In a specific embodiment, R¹ is

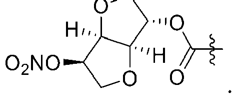

In an embodiment, R² is selected from the group consisting of hydrogen and acyl. In a preferred embodiment, R² is hydrogen. In another preferred embodiment, R² is acetyl.

In an embodiment, R³ is selected from the group consisting of hydrogen, acyl and -L-R'. In a preferred embodiment, R³ is hydrogen. In another preferred embodiment, R³ is acetyl. In another preferred embodiment, R ³ is -L-R', wherein -L- is -(CH₂)ₒ-, where o is 4, one end of - (CH₂)ₒ- is replaced by carbonyl, the other end of -(CH₂)ₒ- is replaced by -O-, and R' is -NO₂ and is connected to -O-. In a specific embodiment, R³ is

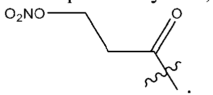

In an embodiment, R⁴ is selected from the group consisting of hydrogen, acyl and -L-R'. In a preferred embodiment, R⁴ is hydrogen. In another preferred embodiment, R⁴ is acetyl. In another preferred embodiment, R⁴ is -L-R', wherein -L- is -(CH₂)ₒ-, where o is 4, one end of - (CH₂)ₒ- is replaced by carbonyl, the other end of -(CH₂)ₒ- is replaced by -O-, and R' is -NO₂ and is connected to -O-. In a specific embodiment, R⁴ is

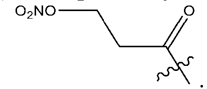

In an embodiment, R⁵ is selected from the group consisting of hydrogen and acyl. In a preferred embodiment, R⁵ is hydrogen. In another preferred embodiment, R⁵ is acetyl. In another preferred embodiment, R⁵ is -L-R', wherein -L- is -(CH₂)ₒ-, where o is 4, one end of - (CH₂)ₒ- is replaced by carbonyl, the other end of -(CH₂)ₒ- is replaced by -O-, and R' is -NO₂ and is connected to -O-. In a specific embodiment, R⁵ is

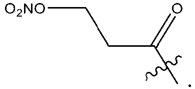

In an embodiment, R⁶ is hydrogen. In an embodiment, R⁷ is hydrogen. In an embodiment, R⁸ is hydrogen. In an embodiment, R⁹ is hydrogen. In an embodiment, R¹⁰ is hydrogen. In an embodiment, R¹¹ is hydrogen. In an embodiment, R¹² is hydrogen. In an embodiment, R¹³ is hydrogen.

In an embodiment, m is 2. In an embodiment, n is 1. In an embodiment, o is 4. When m and n are integers greater than 1, each of R¹⁰, R¹¹, R¹² and R¹³ may be the same or different.

In an embodiment, the chiral carbon atom ^{1*}C has an R configuration. In an embodiment, the chiral carbon atom ^{2*}C has an R or S configuration. In an embodiment, the chiral carbon atom ^{3*}C has an S configuration. In an embodiment, the chiral carbon atom ^{4*}C has an R configuration. In an embodiment, the chiral carbon atom ^{5*}C has an R configuration.

The compounds of the present invention are selected from the group consisting of the following structures: 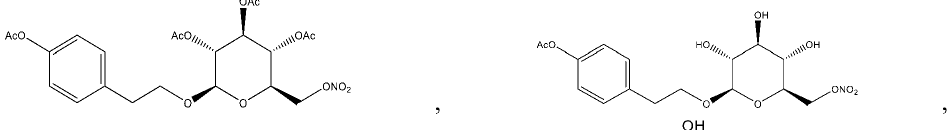 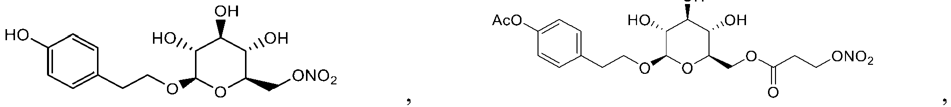 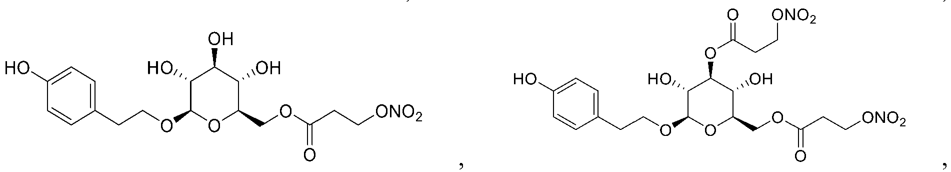 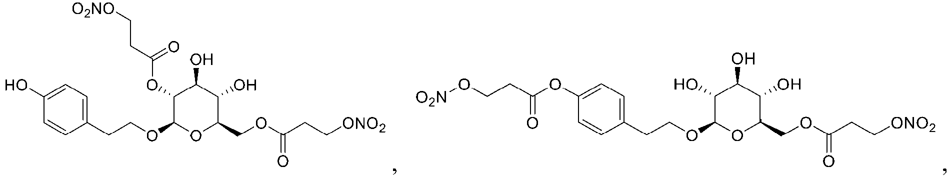 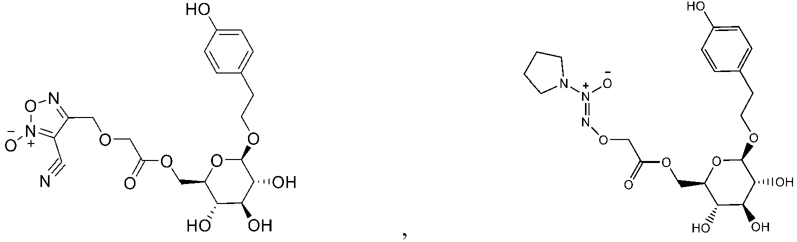 and 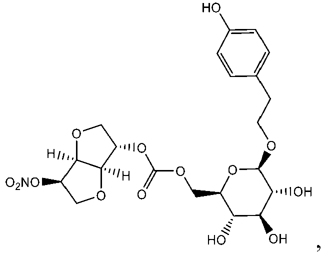 or a pharmaceutically acceptable salt thereof.

In a preferred embodiment, the compound of the present invention is selected from the group consisting of 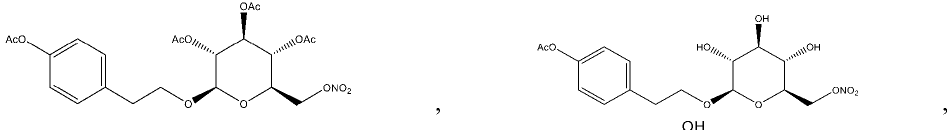 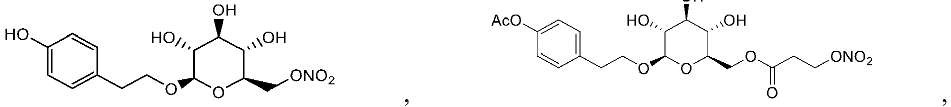 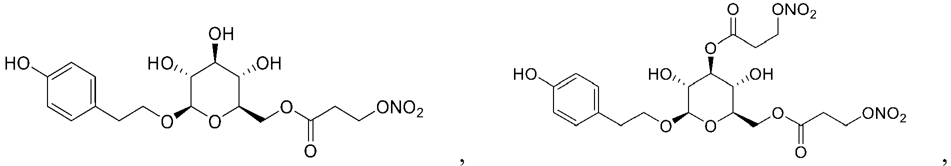 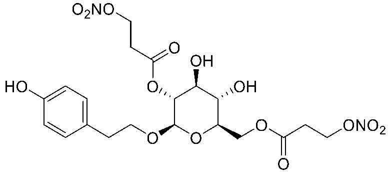 and 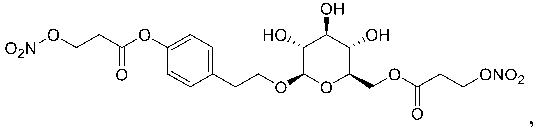 or a pharmaceutically acceptable salt thereof.

In a more preferred embodiment, the compound of the present invention is selected from the group consisting of 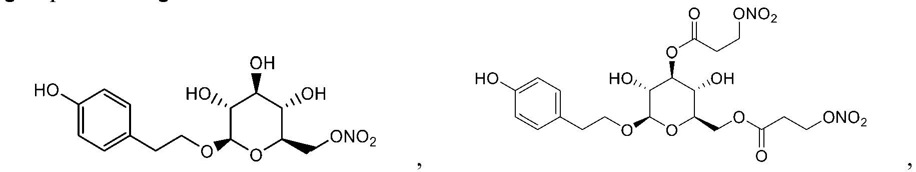 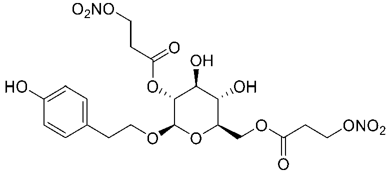 and

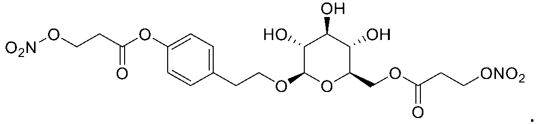

### Pharmaceutically acceptable salts of the compounds of the present invention

Those skilled in the art will appreciate that the compounds according to the present invention may exist in the form of pharmaceutically acceptable salts. As pharmaceutically acceptable salts, for example, the following examples may be provided: metal salts, ammonium salts, salts formed with organic bases, inorganic acids, organic acids, basic or acidic amino acids, and the like. The pharmaceutically acceptable salts according to the present invention can be prepared from compounds containing acidic or basic groups by conventional chemical methods. It can usually be prepared by the reaction of a compound in free acid or base form with a stoichiometrically suitable base or acid in water, an organic solvent or mixtures thereof. In general, non-aqueous media such as ether, ethyl acetate, ethanol, isopropanol, acetonitrile, or the like are preferred.

### Routes of administration, pharmaceutical compositions and kits

The compounds according to the invention will be administered in effective amounts alone or in combination with additional therapeutic agents by any common and acceptable means known in the art. The effective amount may vary depending on the severity of the disease, the age and relative health of the subject, the potency of the compound used, and other factors known to those skilled in the art.

As a general example, a daily dose of about 0.001 to about 100 mg/kg body weight, or particularly about 0.03 to 2.5 mg/kg body weight, may be used. In larger mammals, such as humans, the daily dose may range from about 0.5 mg to about 2000 mg.

The compounds of the present invention are generally administered in the form of a pharmaceutical composition comprising a pharmaceutically active ingredient and various other pharmaceutically acceptable components, see, for example, Remington's Pharmaceutical Science (15th ed., Mack Publishing Company, Easton, Pa., 1980). The preferred or desired form depends on the intended mode of administration and therapeutic application. Depending on the formulation desired, the composition may also include a pharmaceutically acceptable non-toxic carrier or diluent, which is defined as a carrier typically used to formulate pharmaceutical compositions for animal or human administration. The choice of diluent does not affect the biological activity of the composition. Examples of diluents include, but are not limited to, distilled water, physiological phosphate buffered saline, Ringer's solution, dextrose solution, and Hank's solution. In addition, the pharmaceutical composition or formulation may also include other carriers, adjuvants, or non-toxic, non-therapeutic, non-immunogenic stabilizers, etc.

The compounds of the present invention can be administered in the form of pharmaceutical compositions by any conventional route; for example, via the intestinal tract, for example, orally, for example, in the form of tablets, capsules or pills; parenteral, for example, in the form of injectable solutions or suspensions; or topical use, for example, via the eye, nasal cavity, for example, in the form of emulsions, gels, ointments, creams or suppositories.

Therefore, provided is a pharmaceutical composition comprising the compound having the structure of formula (I) or a pharmaceutically acceptable salt thereof according to the present invention, and at least one pharmaceutically acceptable carrier. The compounds according to the present invention may exist in free form combined with at least one pharmaceutically acceptable carrier or in a pharmaceutically acceptable salt form, and can be prepared by conventional methods, for example by mixing, granulating, coating, dissolving or lyophilizing processes.

In an embodiment, the pharmaceutical composition is a solution of the active ingredient, including a suspension or dispersion, such as an isotonic aqueous solution. For the lyophilizate comprising only the active ingredient or the lyophilized composition comprising the active ingredient together with a carrier (such as mannitol), a dispersion or suspension can be prepared prior to use.

Non-limiting examples of carriers include fillers, for example, sugars, such as lactose, sucrose, mannitol or sorbitol, cellulose preparations and/or calcium phosphates, such as tricalcium phosphate or dicalcium phosphate, and binders, such as starches, such as corn, wheat, rice or potato starch, methylcellulose, hydroxypropylmethylcellulose, sodium carboxymethylcellulose and/or polyvinylpyrrolidone, and/or if desired, disintegrants, such as the aforementioned starches, carboxymethyl starch, cross-linked polyvinylpyrrolidone, alginic acid or salts thereof, such as sodium alginate. Other carriers include, but are not limited to, rheology modifiers and lubricants such as silicic acid, talc, stearic acid or salts thereof such as magnesium or calcium stearate, and/or polyethylene glycol or derivatives thereof.

Also provided is a pharmaceutical combination, e.g., a kit, comprising a) a first agent that is a compound having the structure of formula (I) or a pharmaceutically acceptable salt thereof according to the present invention, and b) at least one additional agent. The kit may further comprise instructions for its administration.

### Therapeutic methods and uses of the compounds of the present invention

Provided is a method for treating a disease of abnormal extracellular matrix proteins, such as ocular fibrosis, trabecular meshwork cell and ganglion cell damage, corneal epithelial cell oxidation damage caused by dry eye condition and a disease related to high intraocular pressure, comprising administering to a subject in need thereof a therapeutically effective amount of the compound of formula (I) according to the present invention or a pharmaceutically acceptable salt thereof or the pharmaceutical composition according to the present invention.

Also provided is a compound of formula (I), or a pharmaceutically acceptable salt or pharmaceutical composition thereof, for use in treating, ameliorating or preventing a disease of abnormal extracellular matrix proteins, such as ocular fibrosis, trabecular meshwork cell and ganglion cell damage, corneal epithelial cell oxidation damage caused by dry eye condition and a disease related to high intraocular pressure.

Also provided is use of the compound of formula (I) or a pharmaceutically acceptable salt or pharmaceutical composition thereof in the manufacture of a medicament for treating, ameliorating or preventing a disease of abnormal extracellular matrix proteins, such as ocular fibrosis, trabecular meshwork cell and ganglion cell damage, corneal epithelial cell oxidation damage caused by dry eye condition and a disease related to high intraocular pressure.

In an embodiment, the disease of abnormal extracellular matrix proteins (such as ocular fibrosis, trabecular meshwork cell and ganglion cell damage, corneal epithelial cell oxidation damage caused by dry eye condition) and a disease related to high intraocular pressure include, but are not limited to, glaucoma, subretinal fibrosis in age-related macular degeneration, diabetic retinopathy, high intraocular pressure and tissue fibrosis caused by after cataract.

In embodiment (1), provided is a compound having the following structure of formula (I): 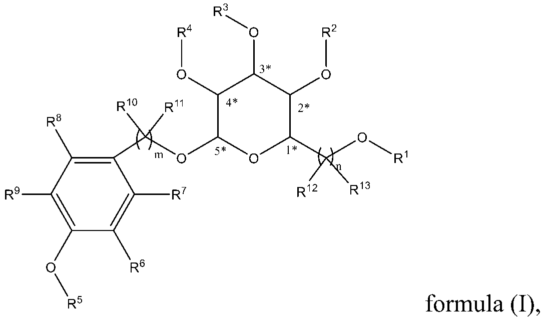
or a pharmaceutically acceptable salt thereof,
wherein,
R¹, R², R³, R⁴ and R⁵ are each independently selected from the group consisting of hydrogen, C₁₋₆ alkyl, C₂₋₆ alkenyl, C₂₋₆ alkynyl, acyl, -NO₂ and -L-R', wherein each alkyl, alkenyl, alkynyl is unsubstituted or substituted with at least one substituent independently selected from the group consisting of R^{X};
-L- is -(CH₂)ₒ-, wherein each -(CH₂)- is independently and optionally replaced by carbonyl or -O-;
R' is selected from the group consisting of hydrogen, C₁₋₆ alkyl, C₂₋₆ alkenyl, C₂₋₆ alkynyl and NO₂, wherein each alkyl, alkenyl, alkynyl is unsubstituted or substituted with at least one substituent independently selected from the group consisting of R^{X};
wherein at least one of R¹, R², R³, R⁴, R⁵ and R' is NO₂;
R⁶, R⁷, R⁸ and R⁹ are each independently selected from the group consisting of hydrogen, C₁₋₆ alkyl, C₁₋₆ alkyl-O-C₁₋₆ alkyl, and C₁₋₆ alkyl-S-C₁₋₆ alkyl, wherein each alkyl is unsubstituted or substituted with at least one substituent independently selected from the group consisting of R^{X};
R¹⁰, R¹¹, R¹² and R¹³ are each independently selected from the group consisting of hydrogen, C₁₋₆ alkyl, C₁₋₆ alkyl-O-C₁₋₆ alkyl, and C₁₋₆ alkyl-S-C₁₋₆ alkyl, wherein each alkyl is unsubstituted or substituted with at least one substituent independently selected from the group consisting of R^{X};
R^{X} is selected from the group consisting of hydroxyl, halogen, C₁₋₆ alkyl, C₁₋₆ alkoxy, C₂₋₆ alkenyl and C₂₋₆ alkynyl, wherein each alkyl, alkoxy, alkenyl, alkynyl is unsubstituted or substituted with at least one substituent independently selected from the group consisting of R^{Y};
R^{Y} is selected from the group consisting of hydroxyl, halogen, C₁₋₆ alkyl, C₁₋₆ alkoxy, C₂₋₆ alkenyl and C₂₋₆ alkynyl;
^{1*}C, ^{2*}C, ^{3*}C, ^{4*}C and ^{5*}C respectively represent the carbon atoms which may have chirality in the compound having the structure of formula (I);
o, m, and n are each independently selected from the group consisting of an integer of 0-6.

In another embodiment (2), provided is the compound of formula (I) or the pharmaceutically acceptable salt thereof according to embodiment (1), wherein R¹ is selected from the group consisting of -NO₂ and -L-R';
preferably,
R¹ is -NO₂; or
R¹ is -L-R', wherein -L- is -(CH₂)ₒ-, where o is 4, one end of -(CH₂)ₒ- is replaced by carbonyl, the other end of -(CH₂)ₒ- is replaced by -O-, and R' is -NO₂ and is connected to -O-.

In another embodiment (3), provided is the compound of formula (I) or the pharmaceutically acceptable salt thereof according to embodiment (1), wherein R² is selected from the group consisting of hydrogen and acyl;
preferably,
R² is hydrogen; or
R² is acetyl.

In another embodiment (4), provided is the compound of formula (I) or the pharmaceutically acceptable salt thereof according to embodiment (1), wherein R³ is selected from the group consisting of hydrogen, acyl and -L-R';
preferably,
R³ is hydrogen;
R³ is acetyl; or
R³ is -L-R', wherein -L- is -(CH₂)ₒ-, where o is 4, one end of -(CH₂)ₒ- is replaced by carbonyl, the other end of -(CH₂)ₒ- is replaced by -O-, and R' is -NO₂ and is connected to -O-.

In another embodiment (5), provided is the compound of formula (I) or the pharmaceutically acceptable salt thereof according to embodiment (1), wherein R⁴ is selected from the group consisting of hydrogen, acyl and -L-R';
preferably,
R⁴ is hydrogen;
R⁴ is acetyl; or
R⁴ is -L-R', wherein -L- is -(CH₂)ₒ-, where o is 4, one end of -(CH₂)ₒ- is replaced by carbonyl, the other end of -(CH₂)ₒ- is replaced by -O-, and R' is -NO₂ and is connected to -O-.

In another embodiment (6), provided is the compound of formula (I) or the pharmaceutically acceptable salt thereof according to embodiment (1), wherein R⁵ is selected from the group consisting of hydrogen and acyl;
preferably,
R⁵ is hydrogen; or
R⁵ is acetyl.

In another embodiment (7), provided is the compound of formula (I) or the pharmaceutically acceptable salt thereof according to embodiment (1), wherein R⁶, R⁷, R⁸, R⁹, R¹⁰, R¹¹, R¹² and R¹³ are each independently hydrogen.

In another embodiment (8), provided is the compound of formula (I) or the pharmaceutically acceptable salt thereof according to embodiment (1), wherein m is 2.

In another embodiment (9), provided is the compound of formula (I) or the pharmaceutically acceptable salt thereof according to embodiment (1), wherein n is 1.

In another embodiment (10), provided is the compound of formula (I) or the pharmaceutically acceptable salt thereof according to embodiment (1), wherein o is 4.

In another embodiment (11), provided is the compound of formula (I) or the pharmaceutically acceptable salt thereof according to embodiment (1), wherein the chiral carbon atom ^{1*}C has an R configuration.

In another embodiment (12), provided is the compound of formula (I) or the pharmaceutically acceptable salt thereof according to embodiment (1), wherein the chiral carbon atom ^{2*}C has an R or S configuration.

In another embodiment (13), provided is the compound of formula (I) or the pharmaceutically acceptable salt thereof according to embodiment (1), wherein the chiral carbon atom ^{3*}C has an S configuration.

In another embodiment (14), provided is the compound of formula (I) or the pharmaceutically acceptable salt thereof according to embodiment (1), wherein the chiral carbon atom ^{4*}C has an R configuration.

In another embodiment (15), provided is the compound of formula (I) or the pharmaceutically acceptable salt thereof according to embodiment (1), wherein the chiral carbon atom ^{5*}C has an R configuration.

In another embodiment (16), provided is the compound of formula (I) according to embodiment (1), which is selected from the group consisting of the following structures: 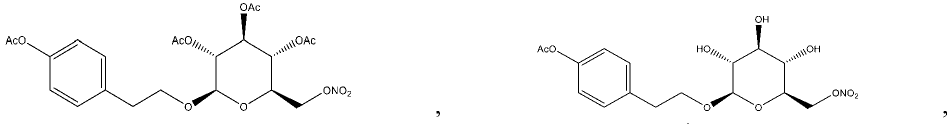 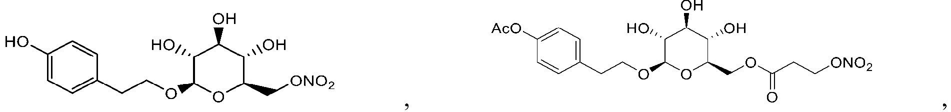 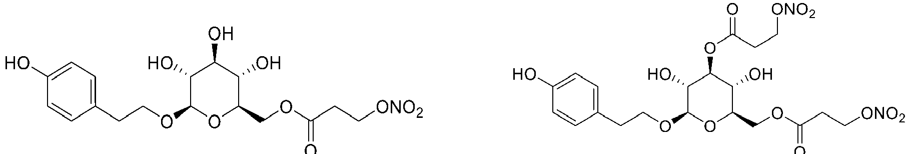 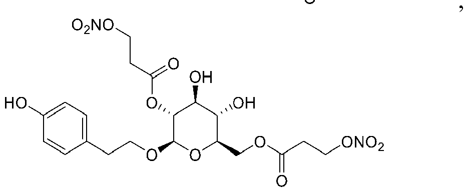 and 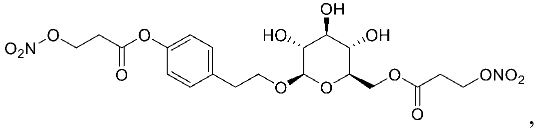 or a pharmaceutically acceptable salt thereof.

In another embodiment (17), provided is a pharmaceutical composition comprising the compound of formula (I) or the pharmaceutically acceptable salt thereof according to any one of embodiments (1) to (16), and at least one pharmaceutically acceptable carrier.

In another embodiment (18), provided is use of the compound of formula (I) or the pharmaceutically acceptable salt thereof according to any one of embodiments (1) to (16) or the pharmaceutical composition according to embodiment (17) in the manufacture of a medicament for treating, ameliorating or preventing a disease of abnormal extracellular matrix proteins and a disease related to high intraocular pressure.

In another embodiment (19), provided is use of the compound of formula (I) or the pharmaceutically acceptable salt thereof according to any one of embodiments (1) to (16) or the pharmaceutical composition according to embodiment (17) in the manufacture of a medicament for preventing or treating glaucoma, subretinal fibrosis in age-related macular degeneration, diabetic retinopathy, high intraocular pressure and tissue fibrosis caused by after cataract.

### Beneficial effects

Provided is a novel compound having the structure of formula (I), which can be used to treat, ameliorate or prevent a disease of abnormal extracellular matrix proteins (such as ocular fibrosis, trabecular meshwork cell and ganglion cell damage, corneal epithelial cell oxidation damage caused by dry eye condition) while also having the effect of reducing intraocular pressure. Therefore, the compound having the structure of formula (I) according to the present invention can be used to treat a disease of abnormal extracellular matrix proteins (for example, pathological mechanisms for aqueous humor outflow obstruction in the trabecular meshwork of glaucoma, subretinal fibrosis in age-related macular degeneration, diabetic retinopathy, tissue fibrosis caused by after cataract, etc.), and can also treat, improve or prevent a disease related to increased intraocular pressure (for example, glaucoma, high intraocular pressure, etc.).

### Examples

The solutions of the present disclosure are further described in detail below with reference to specific embodiments.

It should be noted that the following embodiments are merely examples for clearly illustrating the technical solutions of the present invention, rather than limitations to the present invention. For those of ordinary skill in the art, based on the description of this invention, other variations or changes in different forms can also be made. It is neither necessary nor possible to exhaustively list all the implementation methods here. The obvious variations or changes derived therefrom still fall within the protection scope of this invention. As used herein, instrumentation and reagent materials are commercially available unless otherwise indicated.

### Synthesis Examples

### Example 1 Synthesis of Compound RM-G-003M

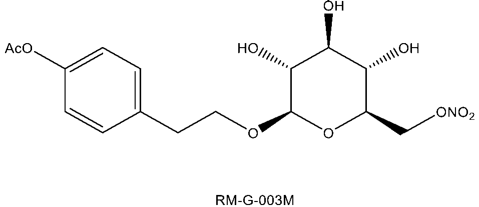

### Synthetic Route:

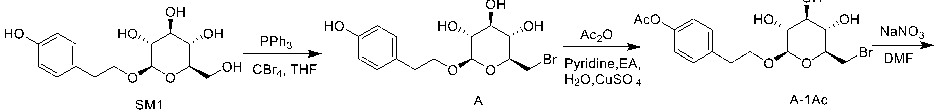

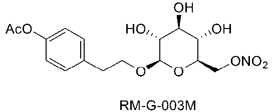

### 1.1 Synthesis of Compound A

The compound SM1 (100 g, 0.33 mol) was dissolved in THF (1 L). Under stirring at room temperature, PPh₃ (1.2 eq.) was added, followed by the portionwise addition of CBr₄ (1.2 eq.). After the addition, the reaction was carried out at room temperature overnight. The mixture was concentrated under reduced pressure to remove THF, and purified by column chromatography to obtain Compound A (30 g, yield 25%) as an oil.

ESI-MS m/z 363.04 [M+H]⁺.

¹H NMR (600 MHz, DMSO-d₆) δ 9.18 (s, 1H), 7.04 (d, J = 7.7 Hz, 2H), 6.66 (d, J = 7.7 Hz, 2H), 5.26 (d, J = 4.7 Hz, 1H), 5.10 (dd, J = 12.2, 4.0 Hz, 2H), 4.26 (d, J = 7.7 Hz, 1H), 3.83 (dd, J = 16.4, 8.1 Hz, 1H), 3.74 (d, J = 10.7 Hz, 1H), 3.59 (dt, J = 17.6, 9.2 Hz, 2H), 3.32 (t, J = 7.8 Hz, 1H), 3.17 (s, 1H), 3.01 (ddd, J = 20.3, 13.0, 8.2 Hz, 2H), 2.76 (d, J = 7.3 Hz, 2H).

### 1.2 Synthesis of Compound A-1Ac

Compound A (30 g, 0.083 mol) was dissolved in pyridine (50 ml). Ac₂O( 17 g, 0.17 mol) was added dropwise while maintaining the internal temperature below 30 °C during the addition. After the addition was complete, the mixture was stirred overnight. Then ethyl acetate was added and pyridine was washed off with aqueous copper sulfate. The mixture was concentrated and dried under reduced pressure, and purified by column chromatography to obtain Compound A-1Ac (17 g, yield 51%) as an off-white solid.

ESI-MS m/z 405.05 [M+H]⁺.

¹H NMR (600 MHz, DMSO-d₆) δ 7.31 (d, J = 7.6 Hz, 2H), 7.02 (d, J = 7.6 Hz, 2H), 5.26 (d, J = 5.0 Hz, 1H), 5.11 (dd, J = 23.1, 4.5 Hz, 2H), 4.29 (d, J = 7.6 Hz, 1H), 3.91 (dd, J = 15.8, 7.9 Hz, 1H), 3.72 (dd, J = 22.5, 9.2 Hz, 2H), 3.58 (d, J = 7.0 Hz, 1H), 3.32 (s, 1H), 3.18 (dd, J = 13.4, 8.3 Hz, 1H), 3.10 - 2.96 (m, 2H), 2.89 (t, J = 6.7 Hz, 2H), 2.25 (s, 3H).

### 1.3 Synthesis of Compound RM-G-003 M

Under nitrogen protection, Compound A-1Ac (17 g, 0.042 mol) and NaNO₃ (50 g, 0.59 mol) were dissolved in N, N-dimethylformamide (DMF) (200 ml), heated to 80 °C, and stirred to react for 16 hours. The reaction system was cooled to room temperature, filtered under suction, concentrated and dried under reduced pressure. An appropriate amount of mixed solution of EA/MeOH = 5:1 was added to dissolve the crude product, then the solid was filtered off under suction. The filtrate was concentrated under reduced pressure and dried, and then purified by silica gel column chromatography to obtain Compound RM-G-003M (6.7g, yield 41%) as a pale yellow oil.

ESI-MS m/z 388.12 [M+H]⁺.

¹H NMR (600 MHz, DMSO-d₆) δ 7.27 (d, J = 7.6 Hz, 2H), 7.02 (d, J = 7.6 Hz, 2H), 5.37 (s, 1H), 4.92 (s, 1H), 4.73 (d, J = 5.8 Hz, 1H), 4.53 (s, 1H), 4.22 (d, J = 3.3 Hz, 1H), 4.09 - 3.92 (m, 2H), 3.86 (s, 1H), 3.63 (t, J = 7.1 Hz, 1H), 3.54 (dd, J = 15.6, 7.8 Hz, 1H), 3.43 (t, J = 8.5 Hz, 1H), 2.82 (t, J = 6.8 Hz, 2H), 2.25 (s, 3H).

### Example 2 Synthesis of Compound RM-G-004M

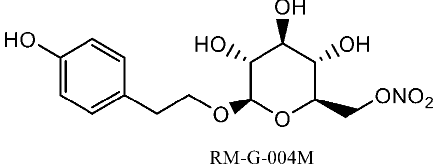

### Synthetic Route:

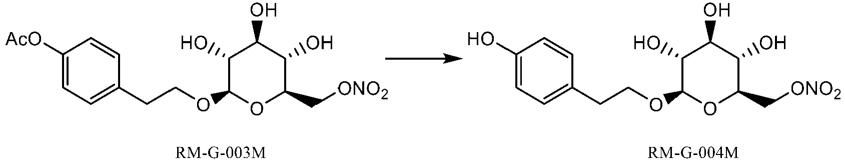

Compound RM-G-003M (6.6 g, 0.017 mol) was dissolved in a mixed solution of methanol (160 ml) and water (6.9 g). K₂CO₃ (4.4 g, 0.032 mol) was added, and the reaction mixture was stirred at room temperature overnight. The reaction of the starting materials was monitored by TLC to show complete reaction. Then the mixture was subjected to suction filtration, washed with an appropriate amount of MeOH, and the pH of the filtrate was adjusted to about 6 using 4N HCl. The solution was concentrated under reduced pressure at 45 °C to obtain a brown solid. Then the product was purified by column chromatography to obtain a pale yellow solid, and further slurried with a mixture solvent of PE/EA = 1:1 to obtain Compound RM-G-004M (3 g, yield 51%) as a white solid.

ESI-MS m/z 346.11[M+H]⁺.

¹H NMR (600 MHz, DMSO-d₆) δ 9.15 (s, 1H), 7.04 - 6.99 (m, 2H), 6.68 - 6.64 (m, 2H), 5.36 (s, 1H), 4.90 (s, 1H), 4.72 (s, 1H), 4.52 (t, J = 4.9 Hz, 1H), 4.21 (d, J = 4.4 Hz, 1H), 4.07 - 4.00 (m, 1H), 3.88 (q, J = 8.2, 7.7 Hz, 1H), 3.85 (s, 1H), 3.64 (t, J = 7.4 Hz, 1H), 3.46 (dd J = 7.9, 7.4 Hz, 2H), 2.69 (t, J = 7.4 Hz, 2H).

### Example 3 Synthesis of Compound RM-G-002M

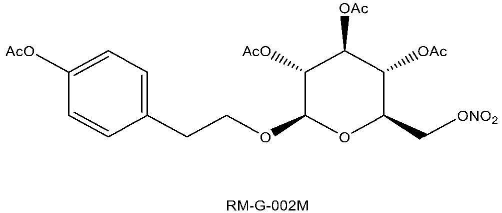

### Synthetic Route:

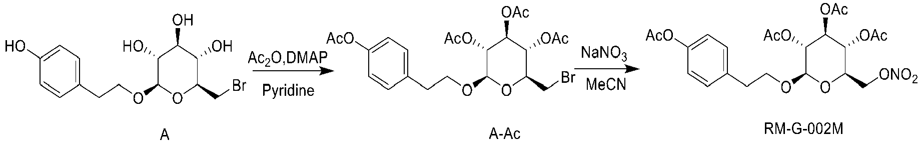

### 3.1 Synthesis of Compound A-Ac

Compound A (10 g, 0.028 mol) was dissolved in pyridine (70 ml). 4-dimethylaminopyridine (DMAP) (30 mg, 0.25 mmol) was added, and the mixture was cooled to an internal temperature of about 0 °C. Then Ac₂O (14 g, 0.14 mol) was added dropwise while maintaining the internal temperature below 5 °C during the addition. After the dropwise addition was completed, the mixture was reacted at room temperature overnight, and the reaction of the starting material Compound A was monitored by TLC to show complete reaction. MeOH (20 ml) was added, stirred for 0.5 hours, and concentrated under reduced pressure to obtain an oil. Ethyl acetate (EA) (100 ml) was then added and the pH of aqueous phase was adjusted to about 2 using 1N HCl. The liquid layers were separated, and the aqueous phase was extracted with EA (100 ml). The organic phases were washed with saturated NaCl. And the organic phases were combined and dried over anhydrous sodium sulfate, concentrated under reduced pressure, and purified by silica gel column chromatography to obtain Compound A-Ac (8 g, yield 54%) as an off-white solid.

ESI-MS m/z: 531.08 [M+H]⁺.

¹H NMR (600 MHz, DMSO-d₆) δ 7.24 (d, J = 7.8 Hz, 2H), 7.02 (d, J = 7.8 Hz, 2H), 5.25 (t, J = 9.5 Hz, 1H), 4.85 (dd, J = 12.5, 8.3 Hz, 2H), 4.76 (t, J = 8.8 Hz, 1H), 4.03 - 3.94 (m, 2H), 3.70 (dd, J = 22.7, 9.2 Hz, 2H), 3.53 (dd, J = 10.8, 6.8 Hz, 1H), 2.83 (s, 2H), 2.25 (s, 3H), 2.01 (s, 3H), 1.92 (s, 3H), 1.87 (s, 3H).

### 3.2 Synthesis of Compound RM-G-002M

Under nitrogen protection, Compound A-Ac (8 g, 0.015 mol) and NaNO₃ (3.8 g, 0.045 mol) were dissolved in MeCN (100 ml), heated to 85 °C, and stirred to react for 72 hours. The reaction system was cooled to room temperature, filtered under suction, concentrated and dried under reduced pressure, and purified by silica gel column chromatography to obtain Compound RM-G-002M (4 g, yield 52%) as a white solid.

ESI-MS m/z 514.15 [M+H]⁺.

¹H NMR (600 MHz, DMSO-d₆) δ 7.22 (d, J = 7.6 Hz, 2H), 7.02 (d, J = 7.7 Hz, 2H), 5.26 (t, J = 9.5 Hz, 1H), 4.88 (dd, J = 19.3, 9.0 Hz, 2H), 4.82 - 4.66 (m, 2H), 4.61 (dd, J = 12.1, 6.0 Hz, 1H), 4.19 - 4.09 (m, 1H), 3.99 - 3.89 (m, 1H), 3.67 (dd, J = 15.4, 7.2 Hz, 1H), 2.80 (s, 2H), 2.25 (s, 3H), 2.01 (s, 3H), 1.94 (s, 3H), 1.87 (s, 3H).

### Example 4 Synthesis of Compound RM-G-005M

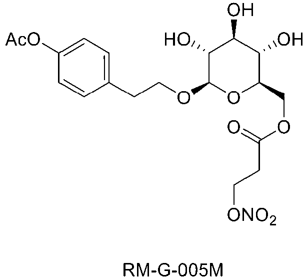

### Synthetic Route:

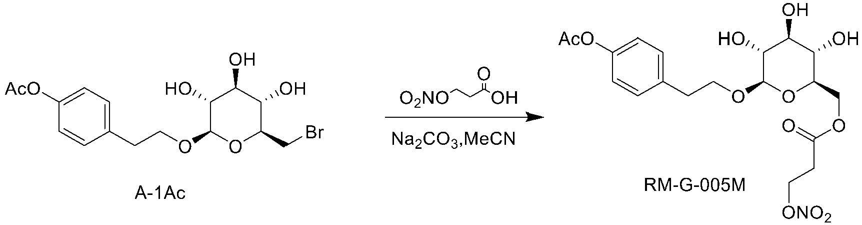

Under nitrogen protection, Compound A-1Ac (8 g, 0.020 mol), 3-(nitrooxy) propanoic acid (6.2 g, 0.046 mol) and Na₂CO₃ (4.9 g, 0.046 mol) were dissolved in MeCN (100 ml), heated to 85 °C, and stirred to react for 72 hours. The reaction system was cooled to room temperature, filtered under suction, concentrated and dried under reduced pressure, and purified by silica gel column chromatography to obtain Compound RM-G-005M (1.8 g, yield 20%) as an oil.

ESI-MS m/z 482.13 [M+Na]⁺.

¹H NMR (600 MHz, DMSO-d₆) δ 7.29 (d, J = 8.0 Hz, 2H), 7.05 - 6.97 (m, 2H), 4.71 (t, J = 6.1 Hz, 2H), 4.34 (d, J = 11.7 Hz, 1H), 4.24 (d, J = 7.8 Hz, 1H), 4.11 (dd, J = 11.9, 6.5 Hz, 1H), 3.92 - 3.85 (m, 1H), 3.71 - 3.65 (m, 1H), 3.36 (dd, J = 9.5, 6.9 Hz, 1H), 3.16 (t, J = 8.8 Hz, 1H), 3.08 (t, J = 9.2 Hz, 1H), 2.97 (t, J = 8.4 Hz, 1H), 2.84 (dq, J = 11.9, 6.2 Hz, 4H), 2.25 (s, 3H), 2.07 (d, J = 1.8 Hz, 2H).

### Example 5 Synthesis of Compound RM-G-006M

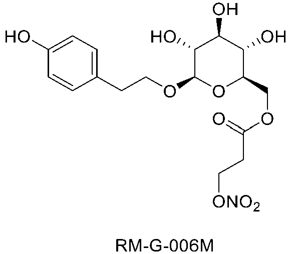

### Synthetic Route:

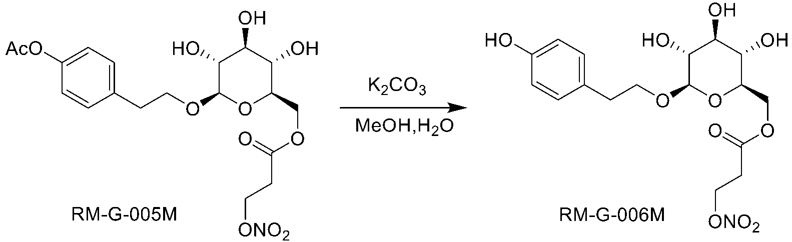

Compound RM-G-005M (3.5 g, 0.0076 mol) was dissolved in a mixed solution of methanol (90 ml) and water (3.9 g), and K₂CO₃ (2.5 g, 0.018 mol) was added. The reaction mixture was stirred at room temperature, and the reaction of the starting material Compound RM-G-005M was monitored by TLC to show complete reaction. The pH of the reaction system was adjusted to about 6 using 4N HCl, concentrated under reduced pressure at 45 °C, and purified by silica gel column chromatography to obtain Compound RM-G-006M (0.5 g, yield 16%) as an oil.

ESI-MS m/z 440.11 [M+Na]⁺.

¹H NMR (600 MHz, DMSO-d₆) δ 9.15 (s, 1H), 7.03 (d, J = 7.8 Hz, 2H), 6.66 (d, J = 7.9 Hz, 2H), 5.40 - 4.90 (m, 3H), 4.71 (t, J = 6.1 Hz, 2H), 4.34 (d, J = 11.7 Hz, 1H), 4.21 (d, J = 7.8 Hz, 1H), 4.11 (dd, J = 11.9, 6.5 Hz, 1H), 3.81 (q, J = 8.3 Hz, 1H), 3.59 (q, J = 8.3 Hz, 1H), 3.35 (dd, J = 9.5, 6.8 Hz, 1H), 3.16 (t, J = 8.9 Hz, 1H), 3.08 (t, J = 9.3 Hz, 1H), 2.97 (t, J = 8.4 Hz, 1H), 2.82 (q, J = 6.1 Hz, 2H), 2.72 (q, J = 6.6 Hz, 2H).

### Example 6 Synthesis of Compounds RM-G-007M and RM-G-008M

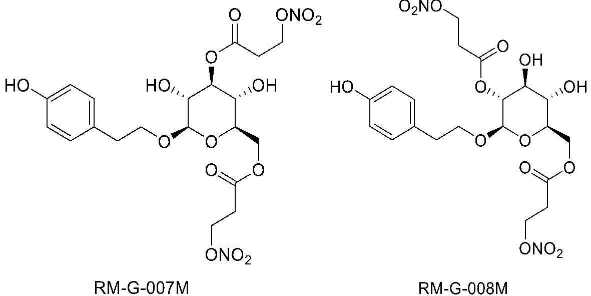

### Synthetic Route:

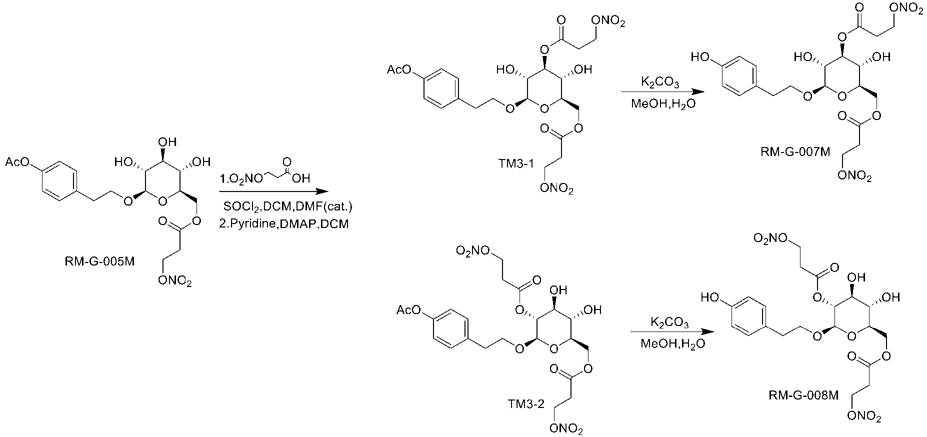

### 6.1 Synthesis of Compounds TM3-1 and TM3-2

Compound 3-(nitrooxy) propanoic acid (2.6 g, 0.019 mol) was dissolved in DCM (8 ml). DMF (0.22 g) was added, and the reaction system was cooled to 0 °C, followed by the dropwise addition of SOCl₂ (2.3 g, 0.019 mol). After the dropwise addition was completed, the reaction was carried out at room temperature for 2 hours to prepare an acyl chloride solution for later use. Compound RM-G-005M (7.5 g, 0.016 mol) was dissolved in DCM (75 ml), pyridine (4.7 g) and DMAP (1.9 g) were added. The reaction system was cooled to -5 °C, then the acyl chloride solution prepared above was added dropwise while maintaining the internal temperature below 0 °C during the addition. After the dropwise addition was completed, the reaction system was heated to room temperature and reacted for 12 hours. Then 1N HCl (45 ml) was added, stirred for 0.5 hours, and the liquid layers were separated. The aqueous phase was extracted with DCM (80 ml). The organic phases were combined, dried over anhydrous sodium sulfate, and concentrated and dried under reduced pressure to obtain Compound TM3-1 (1.2 g, yield 13%) as an oil and Compound TM3-2 (0.6 g, yield 7%) as an oil.

### 6.2 Synthesis of Compounds RM-G-007M and RM-G-008M

Compound TM3-1 (0.5 g, 0.87 mmol) was dissolved in a mixed solution of methanol (10 ml) and water (0.44 g), and K₂CO₃ (0.28 g, 0.0020 mol) was added. The reaction mixture was stirred at room temperature, and the reaction of the starting materials was monitored by TLC to show complete reaction. Then the pH of the reaction system was adjusted to about 6 using 4N HCl, concentrated under reduced pressure at 45 °C, and purified by silica gel column chromatography to obtain Compound RM-G-007M (0.08 g, yield 17%) as an oil.

RM-G-007M: ESI-MS m/z 557.12 [M+Na]⁺.

¹H NMR (600 MHz, DMSO-d₆) δ 9.16 (s, 1H), 6.96 (d, J = 7.7 Hz, 2H), 6.65 (d, J = 7.6 Hz, 2H), 4.70 (dt, J = 21.3, 6.0 Hz, 4H), 4.54 (t, J = 8.8 Hz, 1H), 4.46 (d, J = 8.0 Hz, 1H), 4.35 (d, J = 11.7 Hz, 1H), 4.14 (dd, J = 12.0, 6.4 Hz, 1H), 3.81 (q, J = 7.7 Hz, 1H), 3.53 (q, J = 8.3 Hz, 1H), 3.46 (t, J = 8.0 Hz, 1H), 3.39 (t, J = 9.1 Hz, 1H), 3.20 (t, J = 9.3 Hz, 1H), 2.86 - 2.73 (m, 3H), 2.68 - 2.59 (m, 3H), 2.07 (s, 2H).

Compound TM3-2 (0.5 g, 0.87 mmol) was dissolved in a mixed solution of methanol (10 ml) and water (0.44 g), and K₂CO₃ (0.28 g, 0.0020 mol) was added. The reaction mixture was stirred at room temperature, and the reaction of the starting materials was monitored by TLC to show complete reaction. Then the pH of the reaction system was adjusted to about 6 using 4N HCl, concentrated under reduced pressure at 45 °C, and purified by silica gel column chromatography to obtain Compound RM-G-008M (0.05g, yield 11%) as an oil.

RM-G-008M: ESI-MS m/z 557.12 [M+Na]⁺.

¹H NMR (600 MHz, DMSO-d₆) δ 9.16 (s, 1H), 7.03 (d, J = 8.0 Hz, 2H), 6.66 (d, J = 7.8 Hz, 2H), 4.79 - 4.60 (m, 5H), 4.33 (d, J = 7.8 Hz, 1H), 4.15 (dd, J = 12.2, 5.3 Hz, 1H), 4.05 (d, J = 10.3 Hz, 1H), 3.83 (q, J = 8.3 Hz, 1H), 3.71 - 3.64 (m, 1H), 3.60 (q, J = 9.1, 8.6 Hz, 1H), 3.41 (t, J = 9.2 Hz, 1H), 3.08 (t, J = 8.4 Hz, 1H), 2.89 - 2.63 (m, 6H), 1.24 (s, 1H).

### Example 7 Synthesis of Compound RM-G-009M

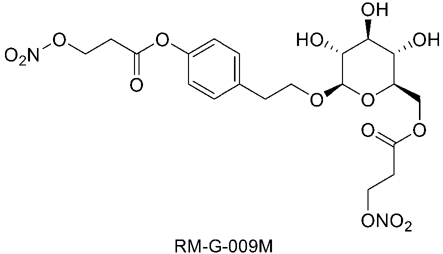

### Synthetic Route:

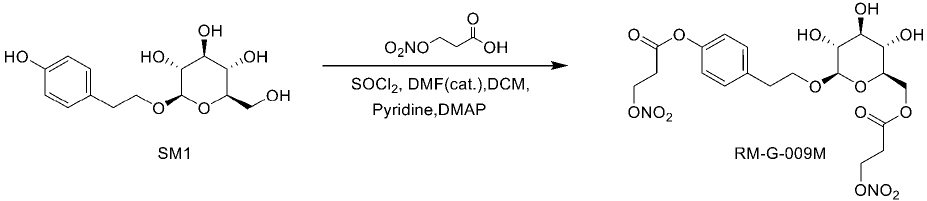

Compound 3-(nitrooxy) propanoic acid (4.0 g, 0.030 mol) was dissolved in DCM (12 ml). DMF (0.34 g) was added, and the reaction system was cooled to 0 °C, followed by the dropwise addition of SOCl₂ (3.5 g, 0.029 mol). After the dropwise addition was completed, the reaction was carried out at room temperature for 2 hours to prepare an acyl chloride solution for later use. SM1 (3.0 g, 0.010 mol) was dissolved in DCM (45 ml), pyridine (2.9 g) and DMAP (1.2 g, 0.0098 mol) were added. The reaction system was cooled to -5°C, then the acyl chloride solution prepared above was added dropwise while maintaining the internal temperature below 0 °C during the addition. After the dropwise addition was completed, the reaction system was heated to room temperature and reacted for 12 hours. Then 1N HCl (28 ml) was added, stirred for 0.5 hours, and the liquid layers were separated. The aqueous phase was extracted with DCM (50 ml). The organic phases were combined, dried over anhydrous sodium sulfate, concentrated and dried under reduced pressure, and purified by silica gel column chromatography to obtain RM-G-009M (0.5 g, yield 10%) as an oil.

ESI-MS m/z 557.12 [M+Na]⁺.

¹H NMR (600 MHz, DMSO-d₆) δ 7.32 (d, J = 7.6 Hz, 2H), 7.04 (d, J = 7.7 Hz, 2H), 5.21 (s, 1H), 5.12 (s, 2H), 4.83 (t, J = 5.5 Hz, 2H), 4.71 (t, J = 5.8 Hz, 3H), 4.34 (d, J = 11.8 Hz, 1H), 4.24 (d, J = 7.3 Hz, 1H), 4.16 - 4.09 (m, 1H), 3.93 - 3.85 (m, 1H), 3.69 (d, J = 8.2 Hz, 1H), 3.11 (t, J = 5.4 Hz, 4H), 2.99 (d, J = 7.8 Hz, 1H), 2.85 (d, J = 6.9 Hz, 4H).

### Example 8 Synthesis of Compound RM-G-010M

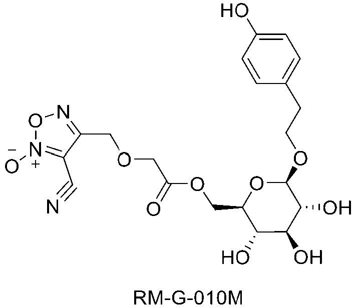

### Synthetic Route:

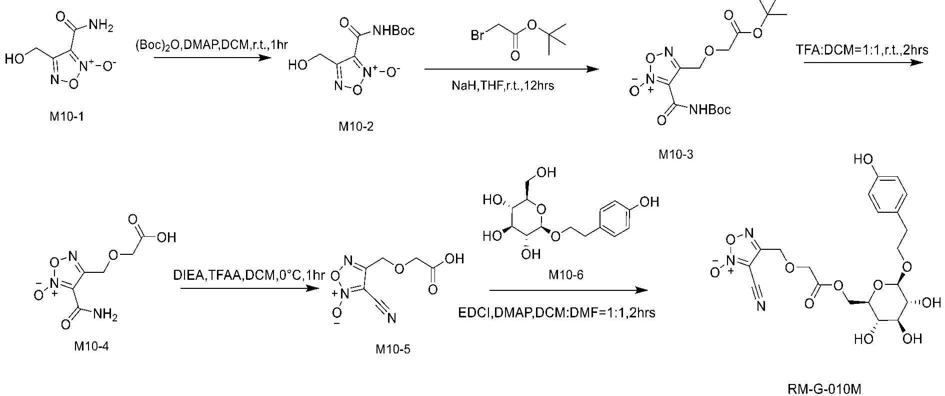

### 8.1 Synthesis of Compound M10-2

Compound M10-1 (1000 mg, 6.28 mmol) was dissolved in dichloromethane (20 mL). (Boc)₂O (1.2 eq.) and DMAP (0.5 eq.) were added, and the mixture was reacted at room temperature for 1 hour. The reaction of the starting material M10-1 was monitored by TLC to show complete reaction. The mixture was washed with an appropriate amount of saturated NaCl. The liquid layers were separated. The organic phases were combined, dried over anhydrous sodium sulfate, concentrated under reduced pressure, and purified by column chromatography (ethyl acetate (EA): petroleum ether (PE) = 1:49-1:9) to obtain Compound M10-2 (1466 mg, yield 90%, purity 96.2%) as a solid.

### 8.2 Synthesis of Compound M10-3

Compound M10-2 (850 mg, 3.28 mmol) and NaH (1 eq.) were dissolved in tetrahydrofuran (10 mL). Tert-butyl 2-bromoacetate (959.7 mg, 1.5 eq.) was added, and the mixture was reacted at room temperature for 12 hours. Ethyl acetate (10 mL) was added to the reaction system, washed with 0.1N HCl aqueous solution, the liquid layers were separated. The aqueous phase was extracted with EA (10 mL), the organic phase was washed with saturated NaCl. The organic phases were combined and dried over anhydrous sodium sulfate, concentrated under reduced pressure and purified by column chromatography (EA: PE = 1:49-1:9) to obtain compound M10-3 (911 mg, yield 74.3%%, purity 97.1%).

### 8.3 Synthesis of Compound M10-4

Compound M10-3 (900 mg, 2.41 mmol) was dissolved in a mixed solvent of TFA: DCM = 1:1 (10 mL), and the reaction was stirred at room temperature for 2 hours. Dichloromethane (DCM) (10 mL) and an appropriate amount of saturated sodium bicarbonate aqueous solution were added to the reaction system and stirred for 10 min. After liquid layers were separated, the DCM layer was concentrated under reduced pressure, and EA (10 mL) was added to the concentrated solution; the pH of the aqueous phase was adjusted to about 2 using 1N HCl. And then the aqueous phase was extracted with EA (10 mL), and the extracted organic phase was washed with saturated NaCl. The EA organic phases were combined and dried over anhydrous sodium sulfate, concentrated under reduced pressure and purified by column chromatography to obtain Compound M10-4 (486.39 mg, yield 93%).

### 8.4 Synthesis of Compound M10-5

Compound M10-4 (400 mg, 1.84 mmol) was dissolved in dichloromethane (10 mL). N, N-diisopropylethylamine (DIEA) (1.2 eq.) and trifluoroacetic anhydride (TFAA) (1.2 eq.) were added, and the mixture was reacted under an ice-water bath for 1 hour. DCM (10 mL) and an appropriate amount of saturated sodium bicarbonate aqueous solution were added to the reaction system and stirred for 10 min. After liquid layers were separated, the DCM layer was concentrated under reduced pressure, and EA (10 mL) was added to the concentrated solution; the pH of the aqueous phase was adjusted to about 2 using 1N HCl. And then the aqueous phase was extracted with EA (10 mL), and the extracted organic phase was washed with saturated NaCl aqueous solution. The EA organic phases were combined and dried over anhydrous sodium sulfate, concentrated under reduced pressure and purified by column chromatography to obtain Compound M10-5 (305.9 mg, yield 83.5%, purity 95.4%).

### 8.5 Synthesis of Compound RM-G-010M

Compound M10-5 (200 mg, 1.0 mmol), Compound M10-6 (300 mg, 1.0 mmol), 1-(3-dimethylaminopropyl)-3-ethylcarbodiimide (EDCI) (186.3 mg, 1.2 mmol) and DMAP (122.2 mg, 1.0 mmol) were dissolved in a mixed solvent of dichloromethane: DMF = 1:1 (5 mL), and the mixture was stirred at room temperature for 2 hours. After completion of the reaction, the mixture was washed with an appropriate amount of saturated NaCl aqueous solution. The aqueous phase was extracted with EA (10 mL), and the organic phases were combined and dried over anhydrous sodium sulfate, concentrated under reduced pressure and purified by column chromatography to obtain Compound RM-G-010M (76.5 mg, yield 15.9%, purity 97.2%).

ESI-MS m/z 482.14 [M+H]⁺.

¹H NMR (400 MHz, DMSO-d₆) δ 9.15(s, 1H), δ 7.03 (dt, J = 8.5, 1.0 Hz, 2H), 6.75 - 6.69 (m, 2H), 5.00 (d, J = 6.1 Hz, 1H), 4.76-4.68 (m, 2H), 4.63 (d, J = 1.1 Hz, 2H), 4.34 (dd, J = 11.8, 4.9 Hz, 1H), 4.27-4,20 (m, 3H), 3.90 (d, J = 6.6 Hz, 1H), 3.81 (dt, J = 11.7, 6.4 Hz, 1H), 3.75 - 3.58 (m, 3H), 3.47-3.42 (m, 1H), 3.35-3.31 (m, 1H), 2.88 (tt, J = 6.3, 0.9 Hz, 2H).

### Example 9 Synthesis of Compound RM-G-011M

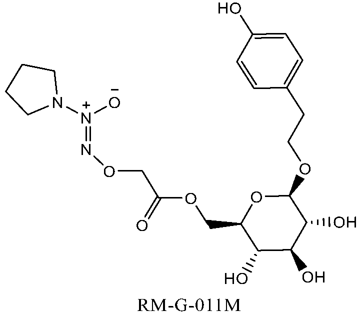

### Synthetic Route:

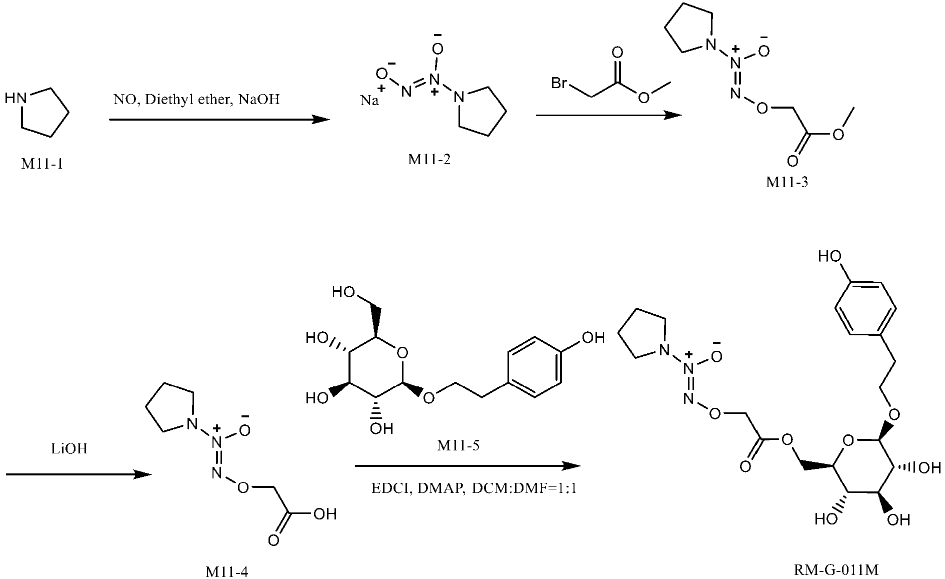

### 9.1 Synthesis of Compound M11-2

Tetrahydropyrrole (10 g, 140.8 mmol) was dissolved in diethyl ether (100 mL). Sodium hydroxide (1.0 eq.) was added. After complete dissolution, the mixture was transferred to a stainless steel high-pressure reactor. The air was replaced with nitrogen, then NO gas was introduced and the pressure was maintained at 15 psi. The reaction was carried out at room temperature for 3 days. After completion of the reaction, the reaction solution was filtered and washed with diethyl ether to obtain Compound M11-2 (21.5g, yield 87%, purity 98.7%).

### 9.2 Synthesis of Compound M11-4

Compound M11-2 (10 g, 65.3 mmol) and methyl bromoacetate (1.2 eq.) were dissolved in methanol (100 mL) and stirred at room temperature for 4 hours. Then lithium hydroxide (1 mol/L aqueous solution, 130 mL) was slowly added and the mixture was reacted at room temperature for 12 hours. The reaction system was concentrated under reduced pressure, the remaining aqueous solution was washed twice with diethyl ether (100 mL), and then the organic phase of diethyl ether was extracted twice with water. The aqueous phase was combined, the pH was adjusted to about 2 using 1N HCl. The resulting solution was extracted with EA (150 mL) three times, and the organic phases of the combined EA were dried over anhydrous sodium sulfate and concentrated to obtain Compound M11-4 (8.51 g, 41.9 mmol, yield 64.2%, purity 98.0%).

### 9.3 Synthesis of Compound RM-G-011M

Compound M11-4 (1 g, 5.3 mmol), Compound M11-5 (1.59 g, 5.3 mmol), EDCI (1.2 eq.) and DMAP (1.0 eq.) were dissolved in a mixed solvent (10 mL) of DCM: DMF = 1:1 to carry out the reaction. After completion of the reaction, the mixture was washed with an appropriate amount of saturated NaCl aqueous solution. The aqueous phase was extracted with EA (10 mL), and the organic phases were combined and dried over anhydrous sodium sulfate, concentrated under reduced pressure and purified by column chromatography to obtain Compound RM-G-011M (443.2 mg, 0.94 mmol, yield 17.7%, purity 98.21%).

ESI-MS m/z 472.19 [M+H]⁺.

¹H NMR (400 MHz, DMSO-d₆) δ 9.16(s, 1H), δ 7.03 (dt, J = 8.5, 1.0 Hz, 2H), 6.79 - 6.66 (m, 2H), 5.00 (d, J = 6.2 Hz, 1H), 4.81 - 4.63 (m, 4H), 4.34 (dd, J = 11.9, 4.9 Hz, 1H), 4.24 (dd, J = 11.7, 4.9 Hz, 1H), 3.90 (d, J = 6.6 Hz, 1H), 3.81 (dt, J = 11.7, 6.4 Hz, 1H), 3.75-3.70 (m, 1H), 3.66-3.58 (m, 6H), 3.47-3.42 (m, 1H), 3.35-3.33 (m, 1H), 2.88 (tt, J = 6.3, 0.9 Hz, 2H), 2.00 - 1.82 (m, 4H).

### Example 10 Synthesis of Compound RM-G-027M

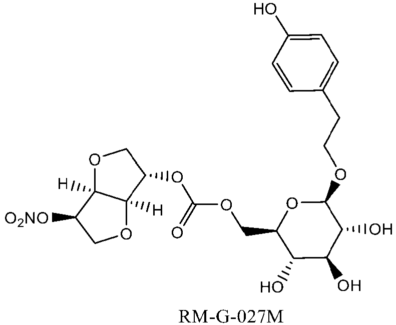

### Synthetic Route:

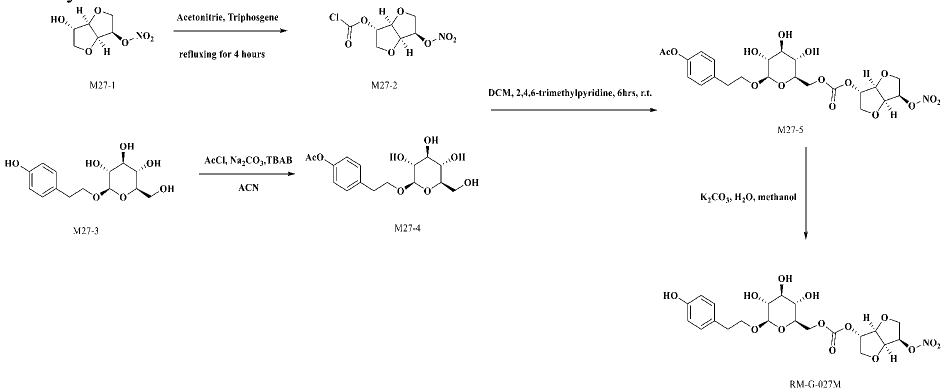

### 10.1 Synthesis of Compound M27-2

Compound M27-1 (5 g, 26.43 mmol) was dissolved in acetonitrile (25 mL). Excessive amount of triphosgene (1.2 eq.) was added, and the reaction was refluxed for 4 hours. After completion of the reaction, the solvent was concentrated and dried under reduced pressure to obtain Compound M27-2 (5.88g).

### 10.2 Synthesis of Compound M27-4

Compound M27-3 (3.00 g, 10 mmol) and sodium carbonate (1.06 g, 10 mmol) were added to an aqueous solution (20 ml) containing tetrabutylammonium bromide (TBAB) (48.4 mg, 0.15 mmol). The mixture was stirred under an ice-water bath, and the temperature of the mixture was cooled to 5°C to obtain the solution for the reaction. Acetyl chloride (10 mmol) was dissolved in ethyl acetate (10 ml), the temperature of the reaction system was maintained at about 5°C under ice-water bath and stirring. And the ethyl acetate solution of acetyl chloride was slowly added to the above solution for the reaction, and the reaction was stirred under ice-water bath for 1 hour. After completion of the reaction, the mixture was extracted with ethyl acetate (15 mL × 3). The organic phases were combined, washed with dilute hydrochloric acid and then separated into layers. The ethyl acetate layer was dried over anhydrous sodium sulfate, concentrated under reduced pressure and purified by column chromatography to obtain Compound M27-4 (2.94g, 8.6 mmol, yield 86%, purity 98.1%).

### 10.3 Synthesis of Compound M27-5

Compound M27-2 (1.98 g, 8 mmol) and Compound M27-4 (2.74 g, 8 mmol) were dissolved in dichloromethane (25 mL). 2,4,6-trimethylpyridine (0.97 g, 1 eq.) was added, and the mixture was stirred at room temperature for 6 hours. After completion of the reaction, the mixture was washed with saturated aqueous sodium bicarbonate solution (12 mL × 3). The dichloromethane layer was dried over anhydrous sodium sulfate, filtered, and the filtrate was concentrated, dried and purified by column chromatography to obtain Compound M27-5 (1.61 g, 2.89 mmol, yield 36.1%, purity 97.8%).

### 10.4 Synthesis of Compound RM-G-027M

Compound M27-5 (1.0 g, 1.8 mmol) was dissolved in methanol (10 ml). Potassium carbonate (0.25 g) was added, and the reaction was stirred at room temperature for 16 h. After completion of the reaction, the mixture was filtered, and the filtrate was purified by column chromatography to obtain Compound RM-G-027M (0.73 g, 1.62 mmol, yield 87.3%, purity 98.3%).

ESI-MS m/z 518.15 [M+H]⁺.

¹H NMR (400 MHz, DMSO-d₆) δ9.18 (s, 1H), 7.03 (dt, J = 8.5, 1.0 Hz, 2H), 6.75 - 6.69 (m, 2H), 5.35-5.33 (m, 1H), 5.18-5.16 (m, 1H), 5.00 (d, J = 6.2 Hz, 1H), 4.75 - 4.66 (m, 3H), 4.65-4.63 (m, 1H), 4.46 (dd, J = 11.9, 4.9 Hz, 1H), 4.40 (dd, J = 11.9, 4.9 Hz, 1H), 4.08 (dd, J = 12.4, 2.5 Hz, 1H), 4.00 (dd, J = 12.3, 4.2 Hz, 1H), 3.90 (d, J = 6.6 Hz, 1H), 3.87 - 3.76 (m, 3H), 3.75 - 3.67 (m, 2H), 3.62 (dt, J = 11.7, 6.3 Hz, 1H), 3.47-3.42 (m, 1H), 3.35-3.30 (m, 1H), 2.88 (tt, J = 6.2, 1.0 Hz, 2H).

### Biological Examples

### Biological Example 1 Study on the regulatory mechanism of the compound of formula (I) of the present application for secretion of glaucoma related factors and trabecular meshwork extracellular matrix

1.1 Cell plating: Human normal trabecular meshwork cells (NTM) (BNCC/BeNa Culture Collection) were cultured with F12 containing 10% fetal bovine serum (FBS) (GIBCO, USA). When human normal trabecular meshwork cells reached 75%-80% confluence in good growth status, the cells were digested with 0.25% pancreatin, inoculated into a 6-well plate with 10⁵ cells per well, and cultured at 37 °C in a 5% CO₂ incubator for 2 days.

1.2 Experimental grouping: the original culture solution was discarded, DMEM/F12 culture solution was added for culture, and the cells were grouped according to the following protocols: human normal trabecular meshwork cells were normally cultured as a blank control group, human normal trabecular meshwork cells were added with dexamethasone (Dr.E) as an experimental control group, human normal trabecular meshwork cells were added with dexamethasone and different concentrations of the compounds (RM-G-002M to RM-G-009M, RM-G-010M, RM-G-011M, RM-G-027M) according to the present invention as an experimental group (the concentrations of dexamethasone in the culture solutions of the experimental control group and the experimental group were both 10⁻⁷ mol/L, 3 parallel wells in each group, and the concentrations of the compounds according to the present invention were 0.1 µM, 1 µM, and 10 µM), and the groups were placed at 37°C in a 5% CO₂ incubator for culturing trabecular meshwork cells for 24 h.

### 1.3 Gene detection of trabecular meshwork extracellular matrix proteins:

### 1.3.1 Total RNA extraction

First, the culture solution was aspirated by using a pipette, then washed twice with PBS, and the supernatant was discarded. Subsequently, 1 ml Trizol was added. After allowing the cells to lyse for 5 minutes, the mixture was pipetted up and down repeatedly and transferred to a 1.5 ml EP tube. 400 µl of chloroform was added to the EP tube, which was then vortexed vigorously. The EP tube was allowed to stand on ice for 5 minutes. In a high-speed centrifuge, the EP tube was centrifuged at 4 °C and 12000 rpm for 25 minutes. The EP tube was taken out, 400 µl of the supernatant was aspirated and transferred to a new EP tube. An equal volume of pre-cooled isopropanol was then added, and the EP tube was gently inverted up and down to mix. The tube was placed in a -20 °C refrigerator and allowed to stand for 4 hours. The EP tube was taken out, placed in a high-speed centrifuge, and centrifuged at 4 °C and 12000 rpm for 15 minutes. The supernatant was discarded, then 1 ml of pre-cooled 75% ethanol prepared with DEPC water was added to each EP tube. The mixture was gently vortexed to wash the precipitate at the bottom, and centrifuged at 4 °C and 8000 rpm for 5 minutes. The supernatant was discarded, the EP tube was air dried in a fume hood, and then an appropriate amount of DEPC water was added to dissolve the precipitate.

### 1.3.2 RNA concentration determination

The RNA concentration was adjusted to 1 µg/µl using DEPC water to ensure the value of OD260 (nucleic acid absorption wavelength)/OD280 (protein absorption wavelength) was between 1.8-2.0, and then the RNA was stored in a subzero 80°C refrigerator and reverse transcribed into cDNA as soon as possible.

### 1.3.3 Reverse transcription

Reverse transcription was performed using the PrimeScript^{™} Reverse Transcription Kit (Takara) in a PCR instrument with the following parameters: 37°C for 15 minutes, followed by 85 °C for 5 seconds (to inactivate the reverse transcriptase). The mixture was centrifuged to homogenize and then placed on ice.

### 1.3.4 Quantitative PCR reaction

The cDNA obtained by reverse transcription of RNA was used as a template for qPCR reaction. The reaction was set up in a 10 µL system as follows: 1 µL of 10-fold diluted cDNA template, 1 µL of primers (0.5 µL forward primer plus 0.5 µL reverse primer), 5 µL of SYBR Green PCR Master Mix (Takara), and 3 µL of ddH₂O. The mixture was prepared on ice and protected from light during reagent addition. After the sample was added, the 384-well plate was placed in a centrifuge, and the parameters of the centrifuge were set to 2000 rpm, 4 °C, centrifuged for 2 minutes, and then placed in a qPCR reaction instrument for reaction. Reaction conditions: 95 °C, 30 seconds for initial denaturation; 95 °C, 20 seconds; 60°C, 34 seconds; 72 °C, 40 seconds. Following 40 cycles of reaction, a melting curve was generated using the following conditions: 95 °C for 15 seconds, 60 °C for 40 seconds, and 95 °C for 15 seconds. Results were analyzed using analysis software Delta Delta CT Relative Quantitation.

**Quantitative PCR Primer Table**

| Genes | | Primer (5' - 3') | SEQ ID No. |
|---|---|---|---|
| FN | Forward | AAGACCAGCAGAGGCATAAGG | 1 |
| | Reverse | TGTAGGGGTCAAAGCACGAG | 2 |
| COL-IV | Forward | AAGGAGATGCTGGTCCTCCA | 3 |
| | Reverse | GAGTCCATTGCGTCCAGGAT | 4 |
| LN | Forward | AGCACTGTGGAGCAGTCAGC | 5 |
| | Reverse | TTGCCTGCCAGTTCATCAAG | 6 |

### 1.4 Data analysis

The effect of the compound according to the present invention on the human trabecular meshwork extracellular matrix induced by dexamethasone was detected by Real time PCR, and the experimental results were shown in Figures 1-11. The results show that dexamethasone can increase the mRNA expression levels of human normal trabecular meshwork extracellular fibronectin (FN), collagen type IV (COL-IV) and laminin (LN), while the addition of the compound according to the present invention can reduce the mRNA expression levels of trabecular meshwork extracellular FN, COL-IV and LN. In the Figures, "*" indicates P < 0.05, and "**" indicates P < 0.01.

### Biological Example 2 Effects of the compounds according to the present invention on intraocular pressure in Beagle Dogs

2.1 Compounds (RM-G-002M to RM-G-009M) according to the present invention were dissolved in physiological saline to obtain a 0.25% solution. Compounds (RM-G-010M, RM-G-011M, and RM-G-027M) according to the present invention were dissolved in physiological saline to obtain a 0.25% solution.

2.2 Beagle dogs (Jiangsu YaDong Laboratory Animals Research Institute Co., Ltd.) weighing about 10-14 Kg and aged about 9-21 months were selected (3 dogs in each group).

2.3 All animals were subjected to physical examination before the trial began, and those showing no obvious abnormalities in the anterior segment of the eye and the fundus were confirmed to be healthy and eligible for the study. Animals that failed the physical examination were excluded or replaced prior to the trial commencement.

2.4 At least 4 weeks of behavioral and conscious training for intraocular pressure (IOP) measurement should be conducted before the trial until stable IOP readings were obtained.

2.5 In the experimental administration stage, they were grouped according to the following protocols: normal control group (normal saline group), positive drug group (Timolol Maleate Eye Drops (Bausch & Lomb), specification: 0.5%), compound group I of the present invention (0.2% RM-G-002M to RM-G-009M solution) and compound group II of the present invention (0.25% RM-G-010M solution, 0.25% RM-G-011M solution, 0.25% RM-G-027M solution). The administration route was ocular instillation into the conjunctival sac. The administration volume was 50 µL/eye/time, and the left and right eyes were instilled respectively twice daily at 10:00 and 17:00 for 15 consecutive days. The first day of administration was recorded as Day 1. During administration, the eyelid was gently retracted to avoid contact with the ocular surface. A pipette was used to carefully aspirate 50 µL of the vehicle or test sample, which was then gently instilled into the conjunctival sac. The eyelids were gently closed several times.

2.6 Intraocular pressure measurement: Intraocular pressure measurement was performed once before the first administration on day 1, 2 h and 4 h after the first administration on day 1, before the first administration on day 2, before the first administration on day 4, before the first administration on day 7, before the first administration on day 13, before the first administration on day 15 and on day 16, respectively; detailed measurement data was shown in Table 1, Table 2, Figure 12 and Figure 13.

**Table 1 Summary of beagle dogs IOP data for compound group I of the present Invention**

| Compounds | Concentration (%) | Mean Baseline (mmHg) | ΔIOP reduction amplitude (mmHg) | Change from Baseline (%) |
|---|---|---|---|---|
| Timolol Maleate | 0.5 | 17.6 | 3.1*** | -17.6 |
| RM-G-002M | 0.2 | 17.7 | 4.3*** | -24.5 |
| RM-G-003M | 0.2 | 18.2 | 4.4*** | -24.2 |
| RM-G-004M | 0.2 | 17.4 | 4.7*** | -27.0 |
| RM-G-005M | 0.2 | 17.8 | 4.3*** | -24.2 |
| RM-G-006M | 0.2 | 17.6 | 4.0*** | -22.7 |
| RM-G-007M | 0.2 | 17.5 | 4.8*** | -27.4 |
| RM-G-008M | 0.2 | 17.8 | 5.1*** | -28.6 |
| RM-G-009M | 0.2 | 18.1 | 5.1*** | -28.2 |

| | | | | |
|---|---|---|---|---|
| Compared with the vehicle group, *** represents p ≤ 0.001. | | | | |

**Table 2 Summary of beagle dogs IOP data for compound group II of the present invention**

| Compounds | Concentration (%) | Mean Baseline (mmHg) | Maximum ΔIOP reduction amplitude (mmHg) | Maximum of Change from Baseline (%) |
|---|---|---|---|---|
| Vehicle | 0 | 18.2 | 0.4 | -2.19 |
| Timolol Maleate | 0.5 | 17.6 | 3.0*** | -17.0 |
| RM-G-010M | 0.25 | 18.4 | 4.9*** | -26.6 |
| RM-G-011M | 0.25 | 18.8 | 5.3*** | -28.2 |
| RM-G-027M | 0.25 | 17.7 | 4.8*** | -27.1 |

| | | | | |
|---|---|---|---|---|
| Compared with the vehicle group, *** represents p ≤ 0.001. | | | | |

As shown in the results in Table 1, Table 2, Figure 12 and Figure 13, the normal control group exhibits no significant change in intraocular pressure throughout the experimental process. The average intraocular pressure of the positive drug group and the compound groups of the present invention (group I and group II) shows significant differences compared with that of the normal control group. The compounds in the compound group (group I and group II) of the present invention can achieve a better intraocular pressure reduction effect at concentrations of 0.2% and 0.25% than that of timolol maleate 0.5% in the positive drug group.

### Biological Example 3 Effect of compound on intraocular pressure in rats

3.1 Compounds RM-G-004M, RM-G-010M, RM-G-011M, RM-G-027M were dissolved in a solvent (10 mM disodium hydrogen phosphate buffer solution (pH = 7.0) containing 0.5% Tween 80 and 0.02% benzalkonium chloride) to prepare a 0.25% solution.

3.2 100 SD rats (Charles River) weighing about 160-180 g and aged about 35 days were selected for modeling test grouping. Using the Random Number Table Method, the experimental rats were randomly divided into a blank control group with 16 rats, a dexamethasone group with 68 rats, and a vehicle group with 16 rats.

3.3 All animals were subjected to physical examination before the trial began, and those showing no obvious abnormalities in the anterior segment of the eye and the fundus were confirmed to be healthy and eligible for the study. Animals that failed the physical examination were excluded or replaced prior to the trial commencement.

3.4 The intraocular pressure of the right eye was measured 7 days, 3 days, and 1 day before the commencement of model establishment in rats, serving as the baseline intraocular pressure values for the animals. After the intraocular pressure (IOP) of SD rats stabilized, Dexamethasone Sodium Phosphate Eye Drops (Xinxiang Huaqing Pharmaceutical Co., Ltd.) were instilled into the right eyes of rats in the dexamethasone group at a fixed dose of 0.02 ml twice daily at 8:30 and 13:30 (with a 5-hour interval between administrations). The vehicle (0.02 ml) was instilled into the right eyes of rats in the vehicle group using the same schedule. The blank control group was only used as a control. Intraocular pressure was measured every 2 days, and systemic and local ocular reactions in the rats were observed. Model establishment was considered successful when the intraocular pressure of the right eye increased to over 20 mmHg, which occurred after approximately 7-10 days.

3.5 In the experimental administration stage, the successfully modeled rats (60 rats) were selected and evenly grouped according to the following protocols: negative control group (high intraocular pressure vehicle group), positive control group (administered with Timolol Maleate Eye Drops (Bausch & Lomb), specification: 0.5%), Compound RM-G-004M, RM-G-010M, RM-G-011M, RM-G-027M test group (administered with 0.25% compound solution). In addition, 10 rats were randomly extracted from the modeling test vehicle group described in 3.4 above in the same manner as the non-model blank control group in the efficacy test stage. The administration route was ocular instillation into the conjunctival sac. The administration volume was 20 µL/eye/time, and the eye were instilled respectively twice daily at 10:00 and 17:00 for 15 consecutive days. The first day of administration was recorded as Day 1. During administration, the eyelid was gently retracted to avoid contact with the ocular surface. A pipette was used to carefully aspirate 20 µL of the vehicle or test sample, which was then gently instilled into the conjunctival sac. The eyelids were gently closed several times. In this experiment, the negative control group (high intraocular pressure vehicle group), the positive control group (administered with Timolol Maleate Eye Drops (Bausch & Lomb), specification: 0.5%), and the Compounds RM-G-004M, RM-G-010M, RM-G-011M, RM-G-027M test groups (administered with 0.25% compound solution) were subjected to instillation treatment with dexamethasone sodium phosphate eye drops on the right eye of rats twice at the daily fixed time every day, as in the modeling period, to maintain the intraocular pressure of the right eye of rats.

3.6 Intraocular pressure measurement: Intraocular pressure measurement was performed once before the first administration on Day 1, before the first administration on Day 2, before the first administration on Day 4, before the first administration on Day 6, before the first administration on Day 8, before the first administration on Day 10, before the first administration on Day 13, before the first administration on Day 15 and on Day 16, respectively; detailed measurement data at the efficacy test stage was shown in Table 3, Figure 14 and Figure 15.

**Table 3. Effects of Compounds on Intraocular Pressure in Rats**

| Compounds | Concentration (%) | Mean Baseline on Day 1 (mmHg) | Mean Baseline on Day 16 (mmHg) | ΔIOP (mmHg) | ΔIOP (%) |
|---|---|---|---|---|---|
| Timolol Maleate | 0.5 | 21.5 | 16.1 | -5.4 | -25.1*** |
| RM-G-004M | 0.25 | 22.3 | 14.2 | -8.1 | -36.3*** |
| RM-G-010M | 0.25 | 24.1 | 14.5 | -9.6 | -39.8*** |
| RM-G-011M | 0.25 | 22.5 | 13.3 | -9.2 | -40.8*** |
| RM-G-027M | 0.25 | 23.2 | 12.8 | -10.4 | -44.8*** |
| Negative Control Group | / | 23.6 | 23.7 | 0.1 | 0.4 |
| Blank Control Group | / | 10.2 | 10.6 | 0.4 | 3.9 |

| | | | | | |
|---|---|---|---|---|---|
| T. Test: compared with the negative control group, *** represents p ≤ 0.001. | | | | | |

As shown in the results in Table 3, Figure 14 and Figure 15, the blank control group and the negative control group exhibit no significant change in intraocular pressure throughout the experimental process. The average intraocular pressure of the positive control group and the compounds RM-G-004M, RM-G-010M, RM-G-011M, RM-G-027M test groups shows significant differences compared with that of the negative control group. The compounds in the test groups of compounds RM-G-004M, RM-G-010M, RM-G-011M, and RM-G-027M can achieve a better intraocular pressure reduction effect at a concentration of 0.25% than that of timolol maleate 0.5% in the positive control group.

The above descriptions are merely specific embodiments of the present invention, and are not intended to limit the patent scope of the present invention. Any equivalent transformation made by using the present invention, or direct or indirect application in other related technical fields, shall be included in the protection scope of the present invention in a similar way.

## Claims

1. A compound having the structure of the following formula (I): 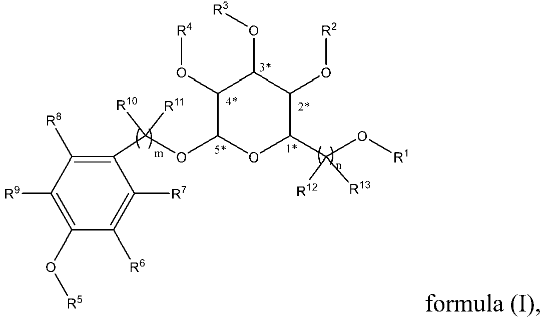
or a pharmaceutically acceptable salt thereof,
wherein,
R¹, R², R³, R⁴ and R⁵ are each independently selected from the group consisting of hydrogen, C₁₋₆ alkyl, C₂₋₆ alkenyl, C₂₋₆ alkynyl, acyl, -NO₂ and -L-R', wherein each alkyl, alkenyl, alkynyl is unsubstituted or substituted with at least one substituent independently selected from the group consisting of R^{X};
-L- is -(CH₂)ₒ-, wherein each -(CH₂)- is independently and optionally replaced by carbonyl or -O-;
R' is selected from the group consisting of hydrogen, C₁₋₆ alkyl, C₂₋₆ alkenyl, C₂₋₆ alkynyl, NO₂, 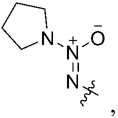 3-10-membered heterocycloalkyl, 5-10-membered heteroaryl, wherein each alkyl, alkenyl, alkynyl, heterocycloalkyl, heteroaryl is unsubstituted or substituted with at least one substituent independently selected from the group consisting of R^{X};
wherein at least one of R¹, R², R³, R⁴, R⁵ and **R'** is NO₂;
R⁶, R⁷, R⁸ and R⁹ are each independently selected from the group consisting of hydrogen, C₁₋₆ alkyl, C₁₋₆ alkyl-O-C₁₋₆ alkyl, and C₁₋₆ alkyl-S-C₁₋₆ alkyl, wherein each alkyl is unsubstituted or substituted with at least one substituent independently selected from the group consisting of R^{X};
R¹⁰, R¹¹, R¹² and R¹³ are each independently selected from the group consisting of hydrogen, C₁₋₆ alkyl, C₁₋₆ alkyl-O-C₁₋₆ alkyl, and C₁₋₆ alkyl-S-C₁₋₆ alkyl, wherein each alkyl is unsubstituted or substituted with at least one substituent independently selected from the group consisting of R^{X};
R^{X} is selected from the group consisting of hydroxyl, halogen, cyano, C₁₋₆ alkyl, C₁₋₆ alkoxy, C₂₋₆ alkenyl, C₂₋₆ alkynyl and 5-10-membered heteroaryl, wherein each hydroxyl, alkyl, alkoxy, alkenyl, alkynyl, heteroaryl is unsubstituted or substituted with at least one substituent independently selected from the group consisting of R^{Y};
R^{Y} is selected from the group consisting of hydroxyl, halogen, cyano, NO₂, C₁₋₆ alkyl, C₁₋₆ alkoxy, C₂₋₆ alkenyl and C₂₋₆ alkynyl;
^{1*}C, ^{2*}C, ^{3*}C, ^{4*}C and ^{5*}C respectively represent the carbon atoms which may have chirality in the compound having the structure of formula (I);
o, m, and n are each independently selected from the group consisting of an integer of 0-6.

2. The compound of formula (I) or the pharmaceutically acceptable salt thereof according to claim 1, wherein
R¹ is selected from the group consisting of -NO₂ and -L-R';
preferably,
R¹ is -NO₂; or
R¹ is -L-R', wherein -L- is -(CH₂)ₒ-, where o is 4, one end of -(CH₂)ₒ- is replaced by carbonyl, the other end of -(CH₂)ₒ- is replaced by -O-, and R' is -NO₂ and is connected to -O-; or
R¹ is 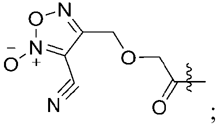 or
R¹ is 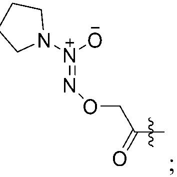 or
R¹ is

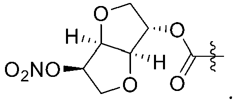

3. The compound of formula (I) or the pharmaceutically acceptable salt thereof according to claim 1, wherein
R² is selected from the group consisting of hydrogen and acyl;
preferably,
R² is hydrogen; or
R² is acetyl.

4. The compound of formula (I) or the pharmaceutically acceptable salt thereof according to claim 1, wherein
R³ is selected from the group consisting of hydrogen, acyl and -L-R';
preferably,
R³ is hydrogen;
R³ is acetyl; or
R³ is -L-R', wherein -L- is -(CH₂)ₒ-, where o is 4, one end of -(CH₂)ₒ- is replaced by carbonyl, the other end of -(CH₂)ₒ- is replaced by -O-, and R' is -NO₂ and is connected to -O-.

5. The compound of formula (I) or the pharmaceutically acceptable salt thereof according to claim 1, wherein
R⁴ is selected from the group consisting of hydrogen, acyl and -L-R';
preferably,
R⁴ is hydrogen;
R⁴ is acetyl; or
R⁴ is -L-R', wherein -L- is -(CH₂)ₒ-, where o is 4, one end of -(CH₂)ₒ- is replaced by carbonyl, the other end of -(CH₂)ₒ- is replaced by -O-, and R' is -NO₂ and is connected to -O-.

6. The compound of formula (I) or the pharmaceutically acceptable salt thereof according to claim 1, wherein
R⁵ is selected from the group consisting of hydrogen and acyl;
preferably,
R⁵ is hydrogen; or
R⁵ is acetyl.

7. The compound of formula (I) or the pharmaceutically acceptable salt thereof according to claim 1, wherein
R⁶, R⁷, R⁸, R⁹, R¹⁰, R¹¹, R¹² and R¹³ are each independently hydrogen.

8. The compound of formula (I) or the pharmaceutically acceptable salt thereof according to claim 1, wherein
m is 2.

9. The compound of formula (I) or the pharmaceutically acceptable salt thereof according to claim 1, wherein
n is 1.

10. The compound of formula (I) or the pharmaceutically acceptable salt thereof according to claim 1, wherein
o is an integer of 2-4.

11. The compound of formula (I) or the pharmaceutically acceptable salt thereof according to claim 1, wherein
the chiral carbon atom ^{1*}C has an R configuration.

12. The compound of formula (I) or the pharmaceutically acceptable salt thereof according to claim 1, wherein
the chiral carbon atom ^{2*}C has an R or S configuration.

13. The compound of formula (I) or the pharmaceutically acceptable salt thereof according to claim 1, wherein
the chiral carbon atom ^{3*}C has an S configuration.

14. The compound of formula (I) or the pharmaceutically acceptable salt thereof according to claim 1, wherein
the chiral carbon atom ^{4*}C has an R configuration.

15. The compound of formula (I) or the pharmaceutically acceptable salt thereof according to claim 1, wherein
the chiral carbon atom ^{5*}C has an R configuration.

16. The compound of formula (I) according to claim 1, which is selected from the group consisting of the following structures: 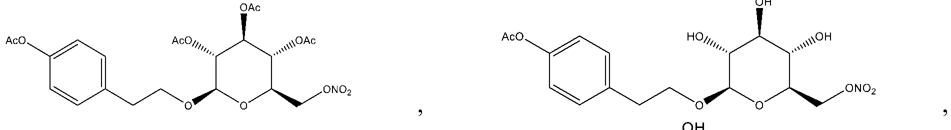 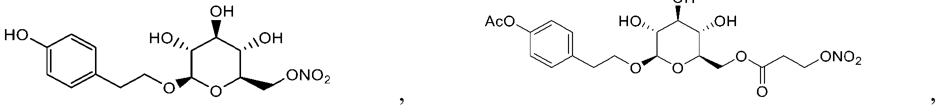 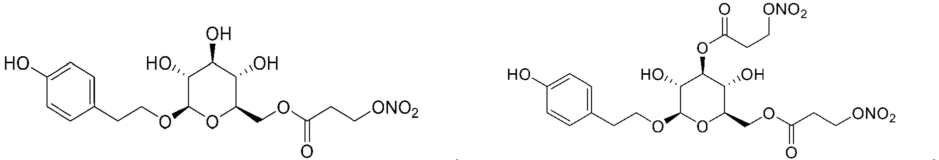 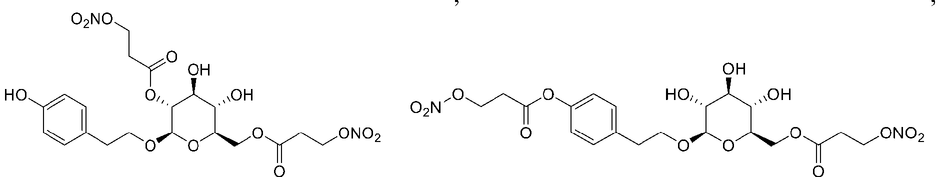 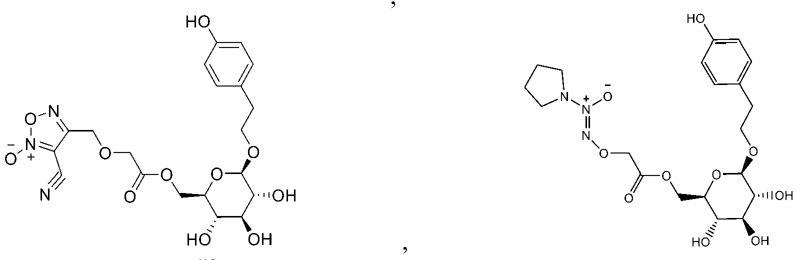 and 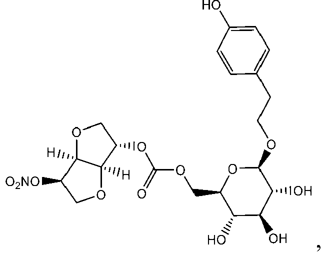 or a pharmaceutically acceptable salt thereof.

17. A pharmaceutical composition comprising the compound of formula (I) or the pharmaceutically acceptable salt thereof according to any one of claims 1 to 16, and at least one pharmaceutically acceptable carrier.

18. Use of the compound of formula (I) or the pharmaceutically acceptable salt thereof according to any one of claims 1 to 16 or the pharmaceutical composition according to claim 17, in the manufacture of a medicament for treating, ameliorating or preventing a disease of abnormal extracellular matrix proteins or a disease related to high intraocular pressure.

19. Use of the compound of formula (I) or the pharmaceutically acceptable salt thereof according to any one of claims 1 to 16 or the pharmaceutical composition according to claim 17, in the manufacture of a medicament for preventing or treating glaucoma, subretinal fibrosis in age-related macular degeneration, diabetic retinopathy, high intraocular pressure and tissue fibrosis caused by after cataract, trabecular meshwork cell and ganglion cell damage, or corneal epithelial cell oxidation damage caused by dry eye condition.
